(19) Europäisches Patentamt
European Patent Office
Office européen des brevets

(11) EP 4 190 332 A1

(12) EUROPEAN PATENT APPLICATION
published in accordance with Art. 153(4) EPC

(43) Date of publication:
**07.06.2023 Bulletin 2023/23**

(21) Application number: **21850852.1**

(22) Date of filing: **28.07.2021**

(51) International Patent Classification (IPC):
***A61K 31/546*** (2006.01) ***A61K 9/19*** (2006.01)
***A61K 47/02*** (2006.01) ***A61K 47/26*** (2006.01)
***A61P 31/04*** (2006.01)

(52) Cooperative Patent Classification (CPC):
**A61K 9/19; A61K 31/546; A61K 47/02; A61K 47/26; A61P 31/04**

(86) International application number:
**PCT/JP2021/027816**

(87) International publication number:
**WO 2022/025091 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.07.2020 JP 2020127763**

(71) Applicant: **Shionogi & Co., Ltd**
**Osaka-shi, Osaka 541-0045 (JP)**

(72) Inventors:
- **KAWASAKI Hidenori**
**Amagasaki-shi, Hyogo 660-0813 (JP)**
- **ITO Masaaki**
**Amagasaki-shi, Hyogo 660-0813 (JP)**

(74) Representative: **Boult Wade Tennant LLP**
**Salisbury Square House**
**8 Salisbury Square**
**London EC4Y 8AP (GB)**

(54) **LYOPHILIZED FORMULATION CONTAINING CEPHALOSPORIN HAVING CATECHOL GROUP, AND METHOD FOR PRODUCING SAME**

(57) The present invention relates to a method for manufacturing a lyophilized formulation which comprises a compound represented by Formula (I):

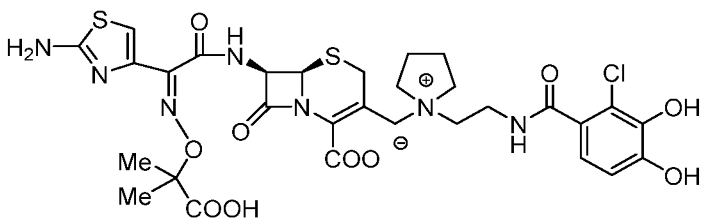

(I)

or its pharmaceutically acceptable salt, wherein the water content is controlled and the reconstitution time is short; and a lyophilized formulation.

With a method for manufacturing a lyophilized formulation comprising at least the following steps:
1) a step of cooling a liquid comprising the compound represented by Formula (I) or its pharmaceutically acceptable salt in a chamber of a lyophilizer to a predetermined cooling temperature, and
2) a step of introducing ice crystals into the chamber,
and a lyophilized formulation having a specific surface area of 0.6 to 1.1 $m^2/g$, the water content can be 0.5% or less, and the reconstitution time can be 30 seconds or less.

EP 4 190 332 A1

Fig. 1

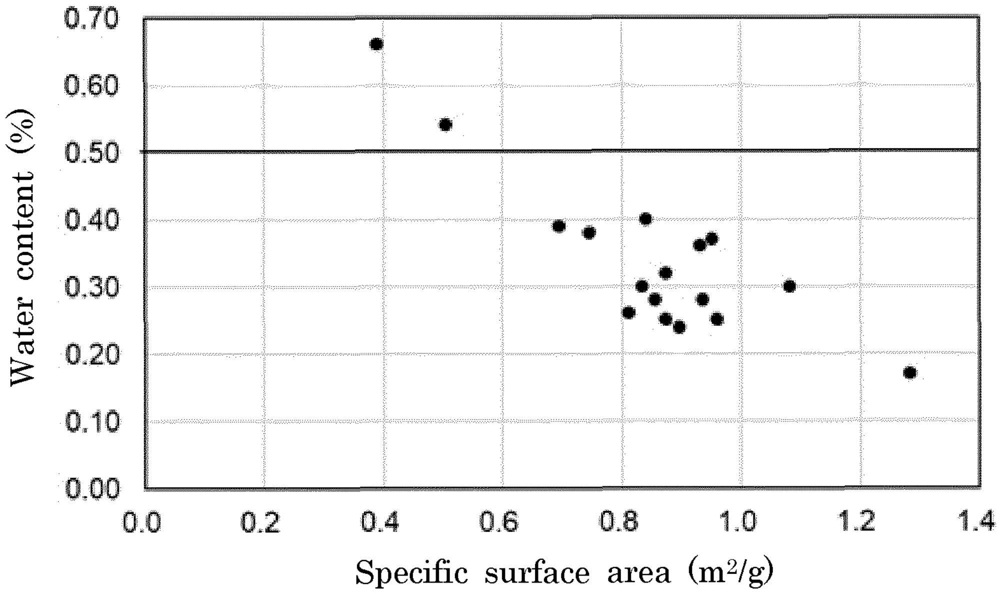
0.70
0.60
0.50
0.40
0.30
0.20
0.10
0.00
Water content (%)
0.0
0.2
0.4
0.6
0.8
1.0
1.2
1.4
Specific surface area (m2/g)

## Description

[TECHNICAL FIELD]

The pharmaceutical formulation of the present invention relates to a lyophilized formulation comprising a cephem compound that has a broad antibacterial spectrum and exhibits strong antibacterial activity especially against β-lactamase-producing Gram-negative bacteria, wherein the water content in the formulation is equal to or less than the water content at which the active ingredient is not decomposed, and the reconstitution time is short.

[BACKGROUND ART]

Various β-lactam medicines have been developed until now, and β-lactam medicines are very important antibacterial medicines clinically. However, the bacterial strains which have acquired tolerance to β-lactam medicines by producing β-lactamase, which degrades β-lactam medicines, are increased. The compound represented by the following Formula (I) or its pharmaceutically acceptable salt was discovered as an agent that exhibits strong antibacterial spectrum against various types of bacteria including Gram-negative bacteria and/or Gram-positive bacteria, and the above problem was solved (Patent Document 1).
Formula (I):

[Chemical formula 1]

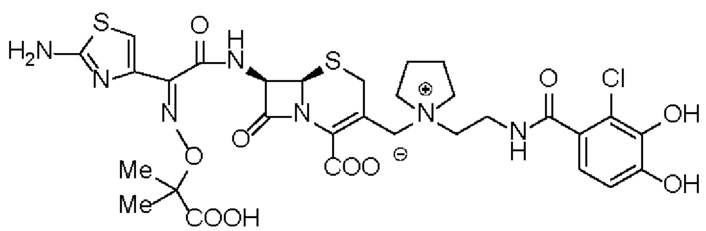

(I)

The compound represented by Formula (I) or its pharmaceutically acceptable salt is used as a lyophilized injection. A lyophilized formulation comprising the compound represented by Formula (I) or its pharmaceutically acceptable salt is disclosed in Patent Document 2.

The manufacturing process of the lyophilized formulation includes: (1) a preliminary freezing step of freeze-solidifying a liquid material to be dried (for example, an aqueous solution comprising a drug or an additive filled in a vial) stored in a drying chamber, (2) a primary drying step of removing water of the material to be dried which has been frozen in the preliminary freezing step, and (3) a secondary drying step of removing a trace amount of non-freezing water captured inside the material to be dried which has become a dry solid through the primary drying step and drying the material to be dried until the water content of the material to be dried reaches a predetermined water content.

In the lyophilization process, the material to be dried in each vial maintains a supercooled state and then forms ice nuclei during the preliminary freezing step (1). Thereafter, the formed ice crystals are sublimated during the primary drying step (2), and non-freezing water and bound water that are not frozen are removed during the secondary drying step (3).

The "supercooling" at the time of the preliminary freezing step refers to a state in which the state of a substance does not change even at a temperature equal to or lower than the temperature at which the substance is to be changed in the phase change of the substance. For example, the "supercooling" is a phenomenon in which a liquid does not solidify and maintains a liquid state even when the liquid is cooled after the temperature of the liquid exceeds the solidifying point. In the case of water, the "supercooling" refers to a state in which water is not frozen even at 0°C or lower, and is in a metastable state, but water rapidly turns into ice when an impact is applied in a supercooled state.

In a case where a substance is in a supercooled state up to a very low temperature, even when freezing starts and ice nuclei are formed, the particle size of the formed ice nuclei is small. The ice nuclei are sublimated through the primary drying step, and the traces of ice nuclei become pores of the lyophilized formulation, that is, pores. Therefore, when the particle size of the ice nucleus is small, the pore size of the lyophilized formulation is also small, and thus even when the lyophilized formulation is reconstituted with water, the water permeation rate in the formulation is slow and the reconstitution time is long.

In recent years, various ice nucleation control techniques have been developed to overcome variations in ice nucleation and ice crystal growth of materials to be dried in the preliminary freezing step. As control of ice nucleation, after the product temperature is controlled to be 0°C or lower, ice crystals (ice fog) are formed using cooled nitrogen

gas and water vapor (pure steam) as raw materials, and the ice fog is introduced into the freeze-drying chamber to induce ice nucleation in the solution, whereby ice nuclei are rapidly formed in all the vials.

In a case where the supercooled state ends at a relatively high temperature, freezing starts, and ice nuclei are formed, the particle size of the formed ice nuclei is larger than the particle size of the ice nuclei that maintained the supercooled state to a very low temperature. Therefore, when the particle size of the ice nucleus is large, the pore size of the lyophilized formulation is also large. Therefore, when the lyophilized formulation is reconstituted with water, the water permeation rate in the formulation is high, and the reconstitution time is short.

Non-Patent Document 1 describes that the freezing temperature can be controlled by using ice fog, and the pore size usually is increased as compared with a normal frozen product, so that the primary drying time can be expected to be shortened. However, this document neither describes nor suggests shortening the reconstitution time of the lyophilized formulation comprising the compound represented by Formula (I) or its pharmaceutically acceptable salt, or reducing the water content of the lyophilized formulation.

Patent Document 3 discloses a lyophilizing method for forming uniform ice nuclei using ice fog. However, Patent Document 3 neither describes nor suggests shortening the reconstitution time of the lyophilized formulation or reducing the water content of the lyophilized formulation. Furthermore, Patent Document 3 neither describes nor suggests shortening the reconstitution time of the lyophilized formulation comprising the compound represented by Formula (I) or its pharmaceutically acceptable salt or reducing the water content of the lyophilized formulation.

[PRIOR ART REFERENCES]

[Non-patent Document]

[Non-patent Document 1] Journal of the Japan Society of Pharmaceutical Machinery and Engineering, 90, vol. 24, No. 2, p. 39 to 51 (2015)

[Patent Document]

[Patent Document 1] International Publication WO 2010/050468A
[Patent Document 2] International Publication WO 2016/035846A
[Patent Document 3] JP 2014-512510 A

[SUMMARY OF THE INVENTION]

[PROBLEMS TO BE SOLVED BY THE INVENTION]

It takes a long time to dissolve the lyophilized formulation disclosed in Patent Document 2 in water (hereinafter, may be referred to as "reconstitution time"), and it takes time to prepare an injectable aqueous solution. In addition, depending on the water content in the formulation, the compound represented by Formula (I) or its pharmaceutically acceptable salt may be decomposed. Therefore, there has been a demand for a lyophilized formulation that comprises the compound represented by Formula (I) or its pharmaceutically acceptable salt, takes a short reconstitution time, and exhibits suppressed decomposition of the compound represented by Formula (I) or its pharmaceutically acceptable salt when the water content in the formulation is set to a certain water content or less.

[MEANS FOR SOLVING THE PROBLEM]

As a result of intensive studies, the present inventors have found that, with a method for manufacturing a lyophilized formulation (hereinafter, may be referred to "manufacturing method of the present invention"), the method including: 1) a step of cooling a liquid comprising a compound represented by Formula (I) or its pharmaceutically acceptable salt in a chamber of a lyophilizer at a predetermined cooling temperature, particularly -30°C to -5°C, and 2) a step of introducing ice crystals into the chamber, the specific surface area is 0.6 to 1.1 $m^2/g$, and the reconstitution time of the lyophilized formulation can be shortened, and moreover, decomposition of the compound represented by Formula (I) or its pharmaceutically acceptable salt can be suppressed by controlling the water content. Furthermore, the present inventors have found that the reconstitution time of the lyophilized formulation can be shortened by designing the specific surface area of the lyophilized formulation comprising the compound represented by Formula (I) or its pharmaceutically acceptable salt to 0.6 to 1.1 $m^2/g$, and moreover, the decomposition of the compound represented by Formula (I) or its pharmaceutically acceptable salt can be suppressed by controlling the water content (hereinafter, the formulation of the

present invention may be referred to as "pharmaceutical formulation of the present invention").

That is, the present invention includes:

(1) A method for manufacturing a lyophilized formulation, which lyophilizes a liquid comprising a compound represented by Formula (I):

[Chemical formula 2]

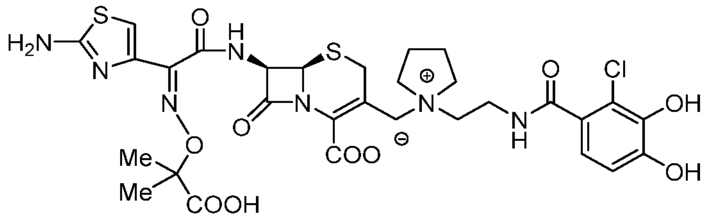

(I)

or its pharmaceutically acceptable salt, the method comprising at least the following steps:

step 1) cooling the liquid comprising the compound represented by Formula (I) or its pharmaceutically acceptable salt in a chamber of a lyophilizer to a predetermined cooling temperature, and
step 2) introducing ice crystals into the chamber.

(2) A method for manufacturing a lyophilized formulation, which comprises the following steps after the step 2),

step 3) further cooling,
step 4) heating and maintaining at a glass transition temperature or higher, and
step 5) drying.

(3) The method for manufacturing a lyophilized formulation according to the above (1) or (2), wherein the liquid comprises at least the following components:

a) the compound represented by Formula (I) or its pharmaceutically acceptable salt,
b) one or more selected from the group consisting of alkali metal chloride, alkali earth metal chloride, transition metal chloride and magnesium chloride, and
c) sugar and/or sugar alcohol.

(4) The method for manufacturing a lyophilized formulation according to the above (1) or (2), wherein the liquid comprises at least the following components:

a) the compound represented by Formula (I) or its pharmaceutically acceptable salt,
b) sodium chloride, and
c) sucrose.

(5) The method for manufacturing a lyophilized formulation according to any one of the above (1) to (4), wherein the step 1) is a step of cooling the liquid to -30°C to -5°C.
(6) The method for manufacturing a lyophilized formulation according to any one of the above (1) to (4), wherein the step 1) is a step of cooling the liquid to -22°C to - 10°C.
(7) The method for manufacturing a lyophilized formulation according to any one of the above (1) to (6), wherein the step 2) is a step of introducing ice crystals.
(8) The method for manufacturing a lyophilized formulation according to any one of the above (2) to (7), wherein the step 5) is a primary drying step and a secondary drying step.
(9) The method for manufacturing a lyophilized formulation according to the above (8), wherein a time of the primary drying step in the step 5) is 100 hours or less.
(10) A lyophilized formulation which comprises the compound represented by Formula (I) or its pharmaceutically acceptable salt, manufactured by the manufacturing method according to any one of the above (1) to (9).
(11) The lyophilized formulation according to the above (10), wherein a specific surface area of the lyophilized formulation is 0.6 to 1.1 $m^2/g$.
(12) A lyophilized formulation which comprises a compound represented by Formula (I):

[Chemical formula 3]

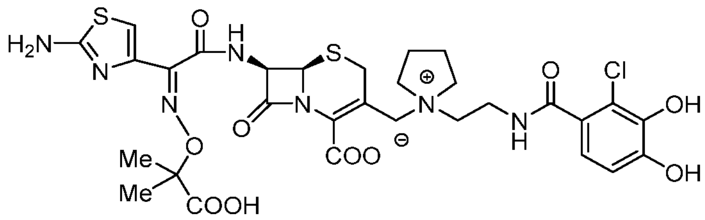

(I)

or its pharmaceutically acceptable salt, wherein a specific surface area of the lyophilized formulation is 0.6 to 1.1 $m^2/g$.

(13) The lyophilized formulation according to the above (11) or (12), wherein a standard deviation of the specific surface area of the lyophilized formulation is 0.2 $m^2/g$ or less.

(14) The lyophilized formulation according to any one of the above (11) to (13), wherein a reconstitution time of the lyophilized formulation is 30 seconds or less.

(15) The lyophilized formulation according to any one of the above (11) to (14), wherein a water content of the lyophilized formulation is 0.5% or less.

(16) The method for manufacturing a lyophilized formulation according to any one of the above (1) to (9), wherein the compound represented by Formula (I) or its pharmaceutically acceptable salt is an amorphous sodium salt of a compound represented by Formula (II):

[Chemical formula 4]

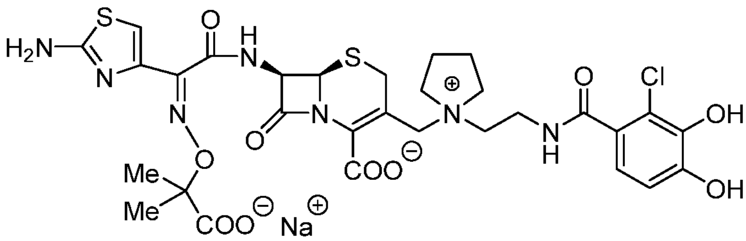

(II)

(17) The lyophilized formulation according to any one of the above (10) to (15), wherein the compound represented by Formula (I) or its pharmaceutically acceptable salt is an amorphous sodium salt of the compound represented by Formula (II).

[EFFECT OF THE INVENTION]

By the manufacturing method of the present invention, a lyophilized formulation comprising a compound represented by Formula (I) or its pharmaceutically acceptable salt and having a specific surface area of 0.6 to 1.1 $m^2/g$ could be manufactured. Furthermore, the reconstitution time could be shortened and the water content could be set to 0.5% or less by preparing a lyophilized formulation comprising the compound represented by Formula (I) or its pharmaceutically acceptable salt and having a specific surface area of 0.6 to 1.1 $m^2/g$. Decomposition of the compound represented by Formula (I) or its pharmaceutically acceptable salt could be suppressed by controlling the water content to this value.

[BRIEF DESCRIPTION OF DRAWINGS]

Fig. 1 shows a relationship between the specific surface area ($m^2/g$) of the lyophilized formulation comprising the compound represented by Formula (I) or its pharmaceutically acceptable salt and the water content (%) of the lyophilized formulation.

[MODE FOR CARRYING OUT THE INVENTION]

Hereinafter, the meaning of each term used in the present specification will be described below. Unless otherwise specified, each term is used in the same meaning when used alone or in combination with another term.

The term "consist of" means having only components.

The term "including" means being not limited to the components, but not excluding elements that are not

described.

Hereinafter, the present invention will be described with reference to embodiments. Throughout the present specification, an expression in a singular form should be understood as also including the concept of its plural form, unless otherwise stated. Therefore, singular articles (for example, "a", "an", "the", and the like in English) should be understood as also including the concept of their plural form, unless otherwise stated.

In addition, the terms used in the present specification should be understood as being used in the meanings commonly used in the art unless otherwise stated. Therefore, unless otherwise defined, all terminology and scientific terms used in the present specification have the same meanings as commonly understood by those skilled in the art to which the present invention belongs. In case of conflict, the present specification (including definitions) prevails.

In the present specification, the active ingredient used in the manufacturing method of the present invention is represented by Formula (I):

[Chemical formula 5]

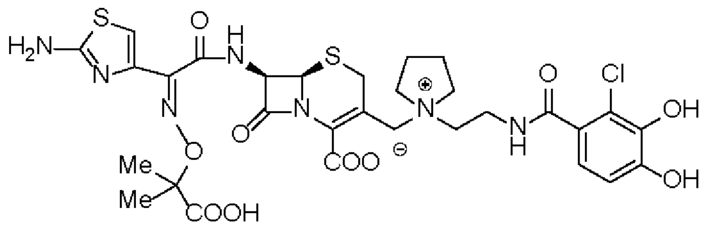

(I)

, but substantially can also be a state of a compound represented by Formula (I'):

[Chemical formula 6]

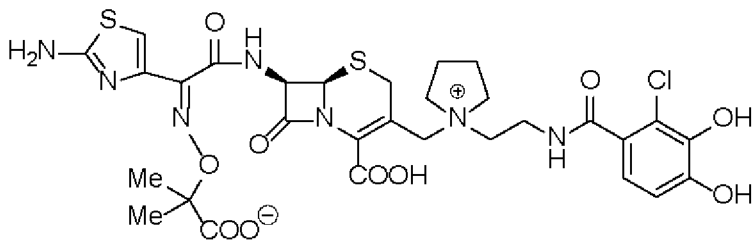

(I')

, so the compounds of the both chemical structures are included in the present invention. The sodium salt of the compound represented by Formula (I) includes

[Chemical formula 7]

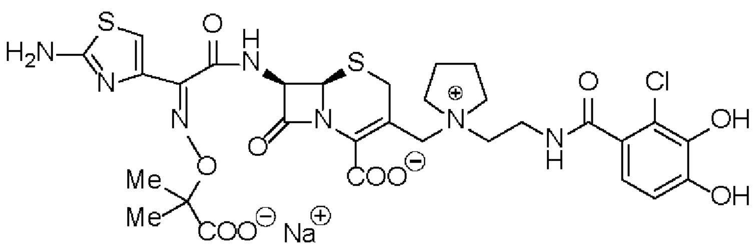

(II)

and

[Chemical formula 8]

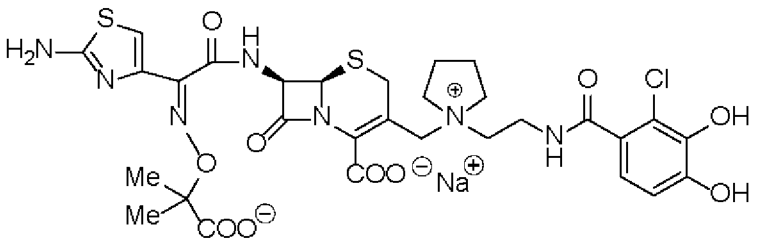

(II')

The compound represented by Formula (I) or its pharmaceutically acceptable salt, and the sodium salt of the compound represented by Formula (II) may be amorphous (non-crystalline). Moreover, the molecular weight of the compound represented by Formula (I) is 752.21 and that of the sodium salt of the compound represented by Formula (II) is 774.20. A method for manufacturing the compound represented by Formula (I) or its pharmaceutically acceptable salt, and the sodium salt of the compound represented by Formula (II) may be the manufacturing methods described in International Publication WO 2010/050468A, International Publication WO 2016/035845A, and International Publication WO 2016/035847A.

The pharmaceutical formulation of the present invention comprises one or more selected from the group consisting of alkali metal chloride, alkali earth metal chloride, transition metal chloride and magnesium chloride, and sugar and/or sugar alcohol to stabilize the compound represented by Formula (I) or its pharmaceutically acceptable salt, and the sodium salt of the compound represented by Formula (II).

As alkali metal chloride, those stabilizing the compound represented by Formula (I) or its pharmaceutically acceptable salt, and the sodium salt of the compound represented by Formula (II), and described in Japanese Pharmacopoeia, Pharmaceutical Standards outside the Japanese Pharmacopoeia, Japanese Pharmaceutical Excipients and Japanese Standard of Food Additives may be used. Examples of alkali metal chloride include sodium chloride, lithium chloride, potassium chloride and the like, preferably sodium chloride.

As alkali earth metal chloride, those stabilizing the compound represented by Formula (I) or its pharmaceutically acceptable salt, and the sodium salt of the compound represented by Formula (II), and described in Japanese Pharmacopoeia, Pharmaceutical Standards outside the Japanese Pharmacopoeia, Japanese Pharmaceutical Excipients and Japanese Standard of Food Additives may be used. Examples of alkali earth metal chloride include barium chloride, calcium chloride and the like, preferably calcium chloride.

As transition metal chloride, those stabilizing the compound represented by Formula (I) or its pharmaceutically acceptable salt, and the sodium salt of the compound represented by Formula (II), and described in Japanese Pharmacopoeia, Pharmaceutical Standards outside the Japanese Pharmacopoeia, Japanese Pharmaceutical Excipients and Japanese Standard of Food Additives may be used. Examples of transition metal chloride include chromium chloride and zinc chloride and the like, preferably zinc chloride.

Moreover, magnesium chloride may be used to stabilize the compound represented by Formula (I) or its pharmaceutically acceptable salt, and the sodium salt of the compound represented by Formula (II). As magnesium chloride, those described in Japanese Pharmacopoeia, Pharmaceutical Standards outside the Japanese Pharmacopoeia, Japanese Pharmaceutical Excipients and Japanese Standard of Food Additives may be used.

As sugar and/or sugar alcohol, those stabilizing the compound represented by Formula (I) or its pharmaceutically acceptable salt, and the sodium salt of the compound represented by Formula (II), and described in Japanese Pharmacopoeia, Pharmaceutical Standards outside the Japanese Pharmacopoeia, Japanese Pharmaceutical Excipients and Japanese Standard of Food Additives may be used. Specifically, saccharide is a monosaccharide, a disaccharide, or a polysaccharide, preferably glucose or fructose (fruit sugar) as a monosaccharide, lactose, sucrose (white sugar, refined white sugar), trehalose, maltose, or isomaltose as a disaccharide, starch or dextrin as a polysaccharide, xylitol, sorbitol, mannitol, lactitol, or the like as a sugar alcohol, and more preferably sucrose (white sugar, refined white sugar).

Moreover, to stabilize the compound represented by Formula (I) or its pharmaceutically acceptable salt, and the sodium salt of the compound represented by Formula (II), alkali metal salt, alkali earth metal salt, transition metal salt or magnesium salt of organic acid or inorganic acid or its hydrate may be used.

Examples of the organic acid or inorganic acid include p-toluene sulfonic acid, benzene sulfonic acid, sulfuric acid, hydrochloric acid and hydrobromic acid, preferably p-toluenesulfonic acid and sulfuric acid.

Examples of the alkali metal salt, alkali earth metal salt, transition metal salt or magnesium salt of organic acid or inorganic acid or its hydrate include sodium salt, lithium salt, potassium salt, calcium salt, zinc salt magnesium salt, and the like, preferably sodium salt, magnesium salt, more preferably sodium salt. Examples of more preferable salt include sodium p-toluene sulfonate, magnesium p-toluene sulfonate, sodium sulfate and magnesium sulfate. Examples of especially preferable salt include sodium p-toluene sulfonate and sodium sulfate.

Typical examples of the compound represented by Formula (I) or its pharmaceutically acceptable salt include the following compounds.

[Chemical formula 9]

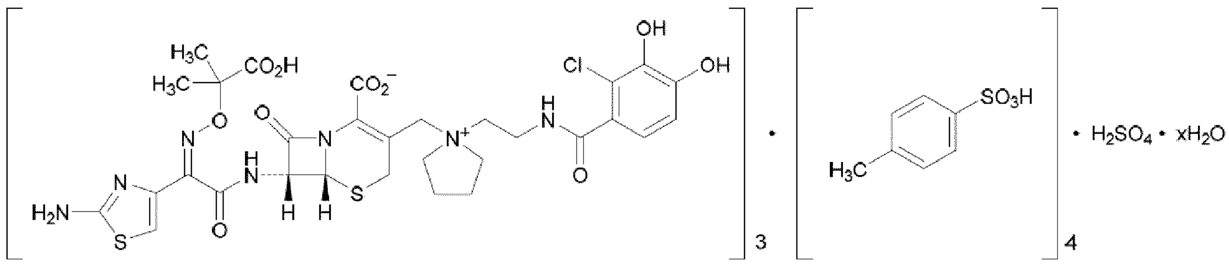

The pharmaceutical formulation may comprise the anti-oxidized agent, buffer agent, soothing agent, preserving agent, and the like which can be used by the injection except those mentioned above, if necessary, those stabilizing the compound represented by Formula (I) or its pharmaceutically acceptable salt, and the sodium salt of the compound represented by Formula (II), described in Japanese Pharmacopoeia, Pharmaceutical Standards outside the Japanese Pharmacopoeia, Japanese Pharmaceutical Excipients and Japanese Standard of Food Additives. Examples of anti-oxidized agent include sodium bisulfite, sodium pyrosulfite, ascorbic acid, and the like. Examples of buffer agent include citric acid salt, acetic acid salt, phosphate acid salt, and the like. Examples of soothing agent include procaine hydrochloride, lidocaine hydrochloride, chlorobutanol, benzyl alcohol, and the like. Examples of preserving agent include methyl parahydroxybenzoate, propyl parahydroxybenzoate, phenol, crezol, benzyl alcohol, chlorobutanol, chlorocrezol, and the like.

As the combination of one or more selected from the group consisting of alkali metal chloride, alkali earth metal chloride, transition metal chloride and magnesium chloride, and sugar and/or sugar alcohol, those that can stabilize the compound represented by Formula (I) or its pharmaceutically acceptable salt, and the sodium salt of the compound represented by Formula (II) may be used.

Examples of these combinations include

a) the compound represented by Formula (I) or its pharmaceutically acceptable salt, sodium chloride and sucrose,
b) the sodium salt of the compound represented by Formula (II), sodium chloride and sucrose,
c) the compound represented by Formula (I) or its pharmaceutically acceptable salt, magnesium chloride and sucrose, and
d) the sodium salt of the compound represented by Formula (II), magnesium chloride and sucrose.

The combination is preferably a) or b), and more preferably b).

When the pharmaceutical preparation comprises sodium chloride as alkali metal chloride and sucrose as sugar and/or sugar alcohol to the compound represented by Formula (I) or its pharmaceutically acceptable salt, a content of sodium chloride is 0.7 to 5.0 molar equivalent, sucrose is 0.3 to 4.0 molar equivalent, preferably, the content of sodium chloride is 1.25 to 4.5 molar equivalent, sucrose is 0.75 to 3.5 molar equivalent, more preferably, the content of sodium chloride is 1.5 to 4.0 molar equivalent, sucrose is 1.0 to 3.0 molar equivalent.

When the pharmaceutical formulation comprises sodium chloride as alkali metal chloride and sucrose as sugar and/or sugar alcohol to sodium salt of the compound represented by Formula (II), a content of sodium chloride is 0.7 to 5.0 molar equivalent, sucrose is 0.3 to 4.0 molar equivalent, preferably, the content of sodium chloride is 1.25 to 4.5 molar equivalent, sucrose is 0.75 to 3.5 molar equivalent, more preferably, the content of sodium chloride is 1.5 to 4.0 molar equivalent, sucrose is 1.0 to 3.0 molar equivalent.

As the combination of one or more selected from the group consisting of alkali metal chloride, alkali earth metal chloride, transition metal chloride and magnesium chloride, sugar and/or sugar alcohol, and alkali metal salt, alkali earth metal salt, transition metal salt or magnesium salt or its hydrate of organic acid or inorganic acid, those stabilizing the compound represented by Formula (I) or its pharmaceutically acceptable acid addition salt or a solvate thereof and the sodium salt of the compound represented by Formula (II) may be used.

Examples of these combinations include

a) the compound represented by Formula (I) or its pharmaceutically acceptable salt, sodium chloride, sucrose and alkali metal salt of p-toluenesulfonic acid,
b) the compound represented by Formula (I) or its pharmaceutically acceptable salt, sodium chloride, sucrose and alkali metal salt of sulfuric acid,

c) the compound represented by Formula (I) or its pharmaceutically acceptable salt, sodium chloride, sucrose, alkali metal salt of p-toluenesulfonic acid and alkali metal salt of sulfuric acid,
d) sodium salt of the compound represented by Formula (II), sodium chloride, sucrose and alkali metal salt of p-toluenesulfonic acid,
e) sodium salt of the compound represented by Formula (II), sodium chloride, sucrose and alkali metal salt of sulfuric acid,
f) sodium salt of the compound represented by Formula (II), sodium chloride, sucrose, alkali metal salt of p-toluenesulfonic acid and alkali metal salt of sulfuric acid,
g) the compound represented by Formula (I) or its pharmaceutically acceptable salt, magnesium chloride, sucrose and alkali metal salt of p-toluenesulfonic acid,
h) the compound represented by Formula (I) or its pharmaceutically acceptable salt, magnesium chloride, sucrose and alkali metal salt of sulfuric acid,
i) the compound represented by Formula (I) or its pharmaceutically acceptable salt, magnesium chloride, sucrose, alkali metal salt of p-toluenesulfonic acid and alkali metal salt of sulfuric acid,
j) sodium salt of the compound represented by Formula (II), magnesium chloride, sucrose and alkali metal salt of p-toluenesulfonic acid,
k) sodium salt of the compound represented by Formula (II), magnesium chloride, sucrose and alkali metal salt of sulfuric acid, and
l) sodium salt of the compound represented by Formula (II), magnesium chloride, sucrose, alkali metal salt of p-toluenesulfonic acid and alkali metal salt of sulfuric acid.

The combination is preferably c) or f), and more preferably f).

When the pharmaceutical formulation comprises sodium chloride as alkali metal chloride, sucrose as sugar and/or sugar alcohol, alkali metal salt of p-toluenesulfonic acid and alkali metal salt of sulfuric acid to the compound represented by Formula (I) or its pharmaceutically acceptable salt, a content of sodium chloride is 0.7 to 5.0 molar equivalent, sucrose is 0.3 to 4.0 molar equivalent, alkali metal salt of p-toluenesulfonic acid salt is 0.25 to 2.5 molar equivalent and alkali metal salt of sulfuric acid is 0.05 to 2.0 molar equivalent, preferably, the content of sodium chloride is 1.25 to 4.5 molar equivalent, sucrose is 0.5 to 4 molar equivalent, alkali metal salt of p-toluenesulfonic acid salt is 0.5 to 2.25 molar equivalent and alkali metal salt of sulfuric acid is 0.075 to 1.5 molar equivalent, more preferably, the content of sodium chloride is 1.5 to 4.0 molar equivalent, sucrose is 1.0 to 3.0 molar equivalent, alkali metal salt of p-toluenesulfonic acid salt is 0.75 to 2.0 molar equivalent and alkali metal salt of sulfuric acid is 0.1 to 1.0 molar equivalent.

When the pharmaceutical formulation comprises sodium chloride as alkali metal chloride, sucrose as sugar and/or sugar alcohol, alkali metal salt of p-toluenesulfonic acid and alkali metal salt of sulfuric acid to the sodium salt of the compound represented by Formula (II), a content of sodium chloride is 0.7 to 5.0 molar equivalent, sucrose is 0.3 to 4.0 molar equivalent, alkali metal salt of p-toluenesulfonic acid is 0.25 to 2.5 molar equivalent and alkali metal salt of sulfuric acid is 0.05 to 2.0 molar equivalent, preferably, the content of sodium chloride is 1.25 to 4.5 molar equivalent, sucrose is 0.5 to 4.0 molar equivalent, alkali metal salt of p-toluenesulfonic acid is 0.5 to 2.25 molar equivalent and alkali metal salt of sulfuric acid is 0.075 to 1.5 molar equivalent, more preferably, the content of sodium chloride is 1.5 to 4.0 molar equivalent, sucrose is 1.0 to 3.0 molar equivalent, alkali metal salt of p-toluenesulfonic acid is 0.75 to 2.0 molar equivalent and alkali metal salt of sulfuric acid is 0.1 to 1.0 molar equivalent.

As the combination of one or more selected from the group consisting of alkali metal chloride, alkali earth metal chloride, transition metal chloride and magnesium chloride, sugar and/or sugar alcohol, and alkali metal salt, alkali earth metal salt, transition metal salt or magnesium salt or its hydrate of organic acid or inorganic acid, those that can stabilize the compound represented by Formula (I) or its pharmaceutically acceptable salt and the sodium salt of the compound represented by Formula (II) may be used.

Examples of these combinations include

a) the compound represented by Formula (I) or its pharmaceutically acceptable salt, sodium chloride, sucrose and sodium p-toluene sulfonate,
b) the compound represented by Formula (I) or its pharmaceutically acceptable salt, sodium chloride, sucrose and sodium sulfate,
c) the compound represented by Formula (I) or its pharmaceutically acceptable salt, sodium chloride, sucrose, sodium p-toluene sulfonate and sodium sulfate,
d) sodium salt of the compound represented by Formula (II), sodium chloride, sucrose and sodium p-toluene sulfonate,
e) sodium salt of the compound represented by Formula (II), sodium chloride, sucrose and sodium sulfate,
f) sodium salt of the compound represented by Formula (II), sodium chloride, sucrose, sodium p-toluene sulfonate and sodium sulfate,

g) the compound represented by Formula (I) or its pharmaceutically acceptable salt, magnesium chloride, sucrose and sodium p-toluene sulfonate,

h) the compound represented by Formula (I) or its pharmaceutically acceptable salt, magnesium chloride, sucrose and sodium sulfate,

i) the compound represented by Formula (I) or its pharmaceutically acceptable acid, magnesium chloride, sucrose, sodium p-toluene sulfonate and sodium sulfate,

j) sodium salt of the compound represented by Formula (II), magnesium chloride, sucrose and sodium p-toluene sulfonate,

k) sodium salt of the compound represented by Formula (II), magnesium chloride, sucrose and sodium sulfate, and

l) sodium salt of the compound represented by Formula (II), magnesium chloride, sucrose, sodium p-toluene sulfonate and sodium sulfate.

The combination is preferably c) or f), and more preferably f).

When the pharmaceutical formulation comprises sodium chloride as alkali metal chloride, sucrose as sugar and/or sugar alcohol, sodium p-toluene sulfonate as alkali metal salt of p-toluenesulfonic acid and sodium sulfate as alkali metal salt of sulfuric acid to the compound represented by Formula (I) or its pharmaceutically acceptable salt, a content of sodium chloride is 0.7 to 5.0 molar equivalent, sucrose is 0.3 to 4.0 molar equivalent, sodium p-toluene sulfonate is 0.25 to 2.5 molar equivalent and sodium sulfate is 0.05 to 2.0 molar equivalent, preferably, the content of sodium chloride is 1.25 to 4.5 molar equivalent, sucrose is 0.5 to 4.0 molar equivalent, sodium p-toluene sulfonate is 0.5 to 2.25 molar equivalent and sodium sulfate is 0.075 to 1.5 molar equivalent, more preferably, the content of sodium chloride is 1.5 to 4.0 molar equivalent, sucrose is 1.0 to 3.0 molar equivalent, sodium p-toluene sulfonate is 0.75 to 2.0 molar equivalent and sodium sulfate is 0.1 to 1.0 molar equivalent.

When the pharmaceutical formulation comprises sodium chloride as alkali metal chloride, sucrose as sugar and/or sugar alcohol, sodium p-toluene sulfonate and sodium sulfate to the sodium salt of the compound represented by Formula (II), a content of sodium chloride is 0.7 to 5.0 molar equivalent, sucrose is 0.3 to 4.0 molar equivalent, sodium p-toluene sulfonate is 0.25 to 2.5 molar equivalent and sodium sulfate is 0.05 to 2.0 molar equivalent, preferably, the content of sodium chloride is 1.25 to 4.5 molar equivalent, sucrose is 0.5 to 4.0 molar equivalent, sodium p-toluene sulfonate is 0.5 to 2.25 molar equivalent and sodium sulfate is 0.075 to 1.5 molar equivalent, more preferably, the content of sodium chloride is 1.5 to 4.0 molar equivalent, sucrose is 1.0 to 3.0 molar equivalent, sodium p-toluene sulfonate is 0.75 to 2.0 molar equivalent and sodium sulfate is 0.1 to 1.0 molar equivalent.

Moreover, sodium gluconate as an additive may be contained.

As the combination of one or more selected from the group consisting of alkali metal chloride, alkali earth metal chloride, transition metal chloride and magnesium chloride, sugar and/or sugar alcohol, and alkali metal salt, alkali earth metal salt, transition metal salt or magnesium salt or its hydrate of organic acid or inorganic acid, and sodium gluconate, those stabilizing the compound represented by Formula (I) or its pharmaceutically acceptable acid addition salt or a solvate thereof and the sodium salt of the compound represented by Formula (II) may be used. Examples of these combinations include

a) the compound represented by Formula (I) or its pharmaceutically acceptable salt, sodium chloride, sucrose, sodium p-toluene sulfonate and sodium gluconate,

b) the compound represented by Formula (I) or its pharmaceutically acceptable salt, sodium chloride, sucrose, sodium sulfate and sodium gluconate,

c) the compound represented by Formula (I) or its pharmaceutically acceptable salt, sodium chloride, sucrose, sodium p-toluene sulfonate, sodium sulfate and sodium gluconate,

d) sodium salt of the compound represented by Formula (II), sodium chloride, sucrose, sodium p-toluene sulfonate and sodium gluconate,

e) sodium salt of the compound represented by Formula (II), sodium chloride, sucrose, sodium sulfate and sodium gluconate,

f) sodium salt of the compound represented by Formula (II), sodium chloride, sucrose, sodium p-toluene sulfonate, sodium sulfate and sodium gluconate,

g) the compound represented by Formula (I) or its pharmaceutically acceptable salt, magnesium chloride, sucrose, sodium p-toluene sulfonate and sodium gluconate,

h) the compound represented by Formula (I) or its pharmaceutically acceptable salt, magnesium chloride, sucrose, sodium sulfate and sodium gluconate,

i) the compound represented by Formula (I) or its pharmaceutically acceptable salt, magnesium chloride, sucrose, sodium p-toluene sulfonate, sodium sulfate and sodium gluconate,

j) sodium salt of the compound represented by Formula (II), magnesium chloride, sucrose, sodium p-toluene sulfonate and sodium gluconate,

k) sodium salt of the compound represented by Formula (II), magnesium chloride, sucrose, sodium sulfate and sodium gluconate, and

l) sodium salt of the compound represented by Formula (II), magnesium chloride, sucrose, sodium p-toluene sulfonate, sodium sulfate and sodium gluconate.

The combination is preferably c) or f), and more preferably f).

When the pharmaceutical formulation comprises sodium chloride as alkali metal chloride, sucrose as sugar and/or sugar alcohol, sodium p-toluene sulfonate as alkali metal salt of p-toluenesulfonic acid and sodium sulfate as alkali metal salt of sulfuric acid to the compound represented by Formula (I) or its pharmaceutically acceptable salt, a content of sodium chloride is 0.7 to 5.0 molar equivalent, sucrose is 0.3 to 4.0 molar equivalent, sodium p-toluene sulfonate is 0.25 to 2.5 molar equivalent, sodium sulfate is 0.05 to 2.0 molar equivalent and sodium gluconate is 0.05 to 1.0 molar equivalent, preferably, the content of sodium chloride is 1.25 to 4.5 molar equivalent, sucrose is 0.5 to 4.0 molar equivalent, sodium p-toluene sulfonate is 0.5 to 2.25 molar equivalent, sodium sulfate is 0.075 to 1.5 molar equivalent and sodium gluconate is 0.075 to 0.75 molar equivalent, more preferably, the content of sodium chloride is 1.5 to 4.0 molar equivalent, sucrose is 1.0 to 3.0 molar equivalent, sodium p-toluene sulfonate is 0.75 to 2.0 molar equivalent, sodium sulfate is 0.1 to 1.0 molar equivalent and sodium gluconate is 0.1 to 0.5 molar equivalent.

When the pharmaceutical formulation comprises sodium chloride as alkali metal chloride, sucrose as sugar and/or sugar alcohol, sodium p-toluene sulfonate and sodium sulfate to the sodium salt of the compound represented by Formula (II), a content of sodium chloride is 0.7 to 5.0 molar equivalent, sucrose is 0.3 to 4.0 molar equivalent, sodium p-toluene sulfonate is 0.25 to 2.5 molar equivalent, sodium sulfate is 0.05 to 2.0 molar equivalent and sodium gluconate is 0.05 to 1.0 molar equivalent, preferably, the content of sodium chloride is 1.25 to 4.5 molar equivalent, sucrose is 0.5 to 4.0 molar equivalent, sodium p-toluene sulfonate is 0.5 to 2.25 molar equivalent, sodium sulfate is 0.075 to 1.5 molar equivalent and sodium gluconate is 0.075 to 0.75 molar equivalent, more preferably, the content of sodium chloride is 1.5 to 4.0 molar equivalent, sucrose is 1.0 to 3.0 molar equivalent, sodium p-toluene sulfonate is 0.75 to 2.0 molar equivalent, sodium sulfate is 0.1 to 1.0 molar equivalent and sodium gluconate is 0.1 to 0.5 molar equivalent.

The pharmaceutical formulation of the present invention is prepared by dissolving or suspending, in water, the compound represented by Formula (I) or its pharmaceutically acceptable salt and the sodium salt of the compound represented by Formula (II) and additives and drying the resulting solution. As the drying method, a drying method stabilizing the compound represented by Formula (I) or its pharmaceutically acceptable salt, and the sodium salt of the compound represented by Formula (II) may be used, and a lyophilizing method is preferable.

The lyophilizing method mainly includes two steps: 1) a step of freezing a sample, and 2) a step of drying the frozen solid.

The lyophilizing method is further subdivided into 1) preliminary freezing, 2) primary drying, and 3) secondary drying as the drying step.

The preliminary freezing is a step of freezing the sample in advance. The water in the sample does not freeze at 0°C because a substance which is not pure water is dissolved therein. Strictly speaking, it is necessary to measure the eutectic point (the temperature at which the sample freezes) and to reliably freeze the sample at the temperature or lower. Usually, a liquid comprising the compound represented by Formula (I) or its pharmaceutically acceptable salt, and an additive is also not frozen in many cases unless the freezing temperature is set to about -40°C or lower. After freezing, ice crystals (crystals of ice) are formed.

The primary drying is a step of sublimating the frozen sample under vacuum. In the primary drying, since water (ice) in the sample is sequentially sublimated from the surface of the sample, it takes time until all the ice is sublimated. Sublimation means that a solid directly turns into a gas without changing to a liquid, or that a gas directly turns into a solid. In the case of lyophilizing, ice crystals (crystals of ice) directly turn into water vapor without changing to water.

Most of the water can be removed at the stage of the primary drying, but the water (bound water) bound between molecules still remains. In order to remove the bound water, a step of heating the sample to a temperature that does not affect the sample is referred to as the secondary drying.

This lyophilizing method of the present invention is performed by a lyophilizer. As the lyophilizer, a vacuum lyophilizer including at least a sample drying unit and a vapor trapping unit (cold trapping unit) can be used, and decompression at the time of vacuum drying is performed by a vacuum pump.

A heat source of the lyophilizer for freezing the sample is necessary for freezing the sample in advance (preliminary freezing). In the case of an apparatus having a large shelf structure, the apparatus has a structure in which a cooling pipe is directly laid on a shelf or a low-temperature heating medium is directly circulated through the shelf. In the case of a laboratory type, preliminary freezing is performed in a low-temperature water bath, a freezer, or the like.

A heat source of the lyophilizer for heating the sample is for promoting sublimation in the drying step and removing bound moisture. An apparatus having a shelf structure has a structure in which a heater or a heated heating medium is directly circulated through the shelf. In a case where lyophilizing is performed in a flask or the like in the laboratory type, room temperature is the heat source.

The vacuum pump sucks air in the lyophilizer and reduces the pressure of the entire lyophilizer to maintain vacuum. Maintaining the vacuum also serves a function of causing the sublimation surface of the sample to self-freeze.
In lyophilizing, in order to allow drying to proceed, it is necessary to constantly remove water vapor generated from the sample and to lower the humidity of the environment where the sample is placed. However, substances have a saturated humidity, and when the environmental humidity increases, evaporation of water vapor is suppressed and drying is stopped. At this time, the cold trapping unit serves a function of discharging water vapor in the apparatus.
The method for manufacturing a pharmaceutical formulation of the present invention is a method for manufacturing a lyophilized formulation, which lyophilizes a liquid comprising a compound represented by Formula (I):

[Chemical formula 10]

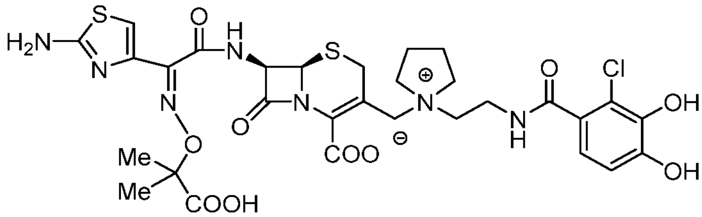

(I)

or its pharmaceutically acceptable salt, the method comprising at least the following steps:

step 1) cooling the liquid comprising the compound represented by Formula (I) or its pharmaceutically acceptable salt in a chamber of a lyophilizer to a predetermined cooling temperature, and
step 2) introducing ice crystals into the chamber.

Another embodiment of the method for manufacturing a pharmaceutical formulation of the present invention is a method for manufacturing a lyophilized formulation, which lyophilizes the liquid comprising the compound represented by Formula (I) or its pharmaceutically acceptable salt, the method comprising at least the following steps:

step 1) cooling the liquid comprising the compound represented by Formula (I) or its pharmaceutically acceptable salt in a chamber of a lyophilizer to a predetermined cooling temperature,
step 2) introducing ice crystals into the chamber, and
step 3) further cooling.

Another embodiment of the method for manufacturing a pharmaceutical formulation of the present invention is a method for manufacturing a lyophilized formulation, which lyophilizes the liquid comprising the compound represented by Formula (I) or its pharmaceutically acceptable salt, the method comprising at least the following steps:

step 1) cooling the liquid comprising the compound represented by Formula (I) or its pharmaceutically acceptable salt in a chamber of a lyophilizer to a predetermined cooling temperature,
step 2) introducing ice crystals into the chamber,
step 3) further cooling, and
step 4) heating and maintaining at a glass transition temperature or higher.

Another embodiment of the method for manufacturing a pharmaceutical formulation of the present invention is a method for manufacturing a lyophilized formulation, which lyophilizes the liquid comprising the compound represented by Formula (I) or its pharmaceutically acceptable salt, the method comprising at least the following steps:

step 1) cooling the liquid comprising the compound represented by Formula (I) or its pharmaceutically acceptable salt in a chamber of a lyophilizer to a predetermined cooling temperature,
step 2) introducing ice crystals into the chamber,
step 3) further cooling,
step 4) heating and maintaining at a glass transition temperature or higher, and
step 5) drying.

Another embodiment of the method for manufacturing a pharmaceutical formulation of the present invention is a method for manufacturing a lyophilized formulation, which lyophilizes a liquid comprising at least the following components:

a) a compound represented by Formula (I):

[Chemical formula 11]

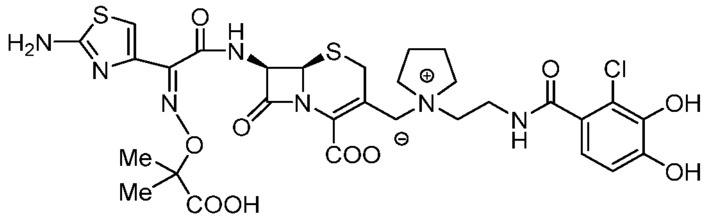

(I)

or its pharmaceutically acceptable salt,
b) one or more selected from the group consisting of alkali metal chloride, alkali earth metal chloride, transition metal chloride and magnesium chloride,
and
c) sugar and/or sugar alcohol,
the method comprising at least the following steps:

step 1) cooling the liquid in a chamber of a lyophilizer to a predetermined cooling temperature, and
step 2) introducing ice crystals into the chamber.

Another embodiment of the method for manufacturing a pharmaceutical formulation of the present invention is a method for manufacturing a lyophilized formulation, which lyophilizes a liquid comprising at least the following components:

a) the compound represented by Formula (I) or its pharmaceutically acceptable salt,
b) one or more selected from the group consisting of alkali metal chloride, alkali earth metal chloride, transition metal chloride and magnesium chloride,
and
c) sugar and/or sugar alcohol,
the method comprising at least the following steps:

step 1) cooling the liquid in a chamber of a lyophilizer to a predetermined cooling temperature,
step 2) introducing ice crystals into the chamber, and
step 3) further cooling.

Another embodiment of the method for manufacturing a pharmaceutical formulation of the present invention is a method for manufacturing a lyophilized formulation, which lyophilizes a liquid comprising at least the following components:

a) the compound represented by Formula (I) or its pharmaceutically acceptable salt,
b) one or more selected from the group consisting of alkali metal chloride, alkali earth metal chloride, transition metal chloride and magnesium chloride,
and
c) sugar and/or sugar alcohol,
the method comprising at least the following steps:

step 1) cooling the liquid in a chamber of a lyophilizer to a predetermined cooling temperature,
step 2) introducing ice crystals into the chamber,
step 3) further cooling, and
step 4) heating and maintaining at a glass transition temperature or higher.

Another embodiment of the method for manufacturing a pharmaceutical formulation of the present invention is a method for manufacturing a lyophilized formulation, which lyophilizes a liquid comprising at least the following components:

a) the compound represented by Formula (I) or its pharmaceutically acceptable salt,

b) one or more selected from the group consisting of alkali metal chloride, alkali earth metal chloride, transition metal chloride and magnesium chloride,
and
c) sugar and/or sugar alcohol,
the method comprising at least the following steps:

step 1) cooling the liquid in a chamber of a lyophilizer to a predetermined cooling temperature,
step 2) introducing ice crystals into the chamber,
step 3) further cooling,
step 4) heating and maintaining at a glass transition temperature or higher, and
step 5) drying.

The method for manufacturing a pharmaceutical formulation of the present invention is a method for manufacturing a lyophilized formulation, which lyophilizes a liquid comprising at least the following components:

a) the compound represented by Formula (I) or its pharmaceutically acceptable salt,
b) sodium chloride,
and
c) sucrose,
the method comprising at least the following steps:

step 1) cooling the liquid in a chamber of a lyophilizer to a predetermined cooling temperature, and
step 2) introducing ice crystals into the chamber.

Another embodiment of the method for manufacturing a pharmaceutical formulation of the present invention is a method for manufacturing a lyophilized formulation, which lyophilizes a liquid comprising at least the following components:

a) the compound represented by Formula (I) or its pharmaceutically acceptable salt,
b) sodium chloride,
and
c) sucrose,
the method comprising at least the following steps:

step 1) cooling the liquid in a chamber of a lyophilizer to a predetermined cooling temperature,
step 2) introducing ice crystals into the chamber, and
step 3) further cooling.

Another embodiment of the method for manufacturing a pharmaceutical formulation of the present invention is a method for manufacturing a lyophilized formulation, which lyophilizes a liquid comprising at least the following components:

a) the compound represented by Formula (I) or its pharmaceutically acceptable salt,
b) sodium chloride,
and
c) sucrose,
the method comprising at least the following steps:

step 1) cooling the liquid in a chamber of a lyophilizer to a predetermined cooling temperature,
step 2) introducing ice crystals into the chamber,
step 3) further cooling, and
step 4) heating and maintaining at a glass transition temperature or higher.

Another embodiment of the method for manufacturing a pharmaceutical formulation of the present invention is a method for manufacturing a lyophilized formulation, which lyophilizes a liquid comprising at least the following components:

a) the compound represented by Formula (I) or its pharmaceutically acceptable salt,

b) sodium chloride,
and
c) sucrose,
the method comprising at least the following steps:

step 1) cooling the liquid in a chamber of a lyophilizer to a predetermined cooling temperature,
step 2) introducing ice crystals into the chamber,
step 3) further cooling,
step 4) heating and maintaining at a glass transition temperature or higher, and
step 5) drying.

The method for manufacturing a pharmaceutical formulation of the present invention is a method for manufacturing a lyophilized formulation, which lyophilizes a liquid comprising sodium salt of a compound represented by Formula (II):

[Chemical formula 12]

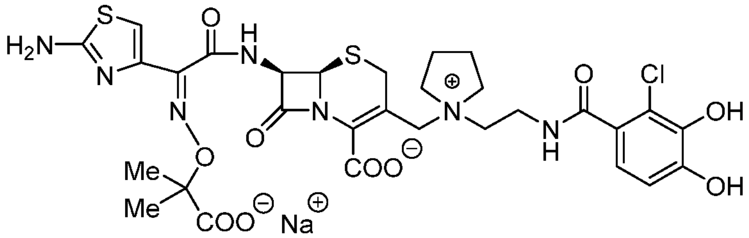

(II)

.

the method comprising at least the following steps:

step 1) cooling the liquid comprising sodium salt of the compound represented by Formula (II) in a chamber of a lyophilizer to a predetermined cooling temperature, and
step 2) introducing ice crystals into the chamber.

Another embodiment of the method for manufacturing a pharmaceutical formulation of the present invention is a method for manufacturing a lyophilized formulation, which lyophilizes the liquid comprising sodium salt of the compound represented by Formula (II), the method comprising at least the following steps:

step 1) cooling the liquid comprising the sodium salt of the compound represented by Formula (II) in a chamber of a lyophilizer to a predetermined cooling temperature,
step 2) introducing ice crystals into the chamber, and
step 3) further cooling.

Another embodiment of the method for manufacturing a pharmaceutical formulation of the present invention is a method for manufacturing a lyophilized formulation, which lyophilizes the liquid comprising sodium salt of the compound represented by Formula (II), the method comprising at least the following steps:

step 1) cooling the liquid comprising the sodium salt of the compound represented by Formula (II) in a chamber of a lyophilizer to a predetermined cooling temperature,
step 2) introducing ice crystals into the chamber,
step 3) further cooling, and
step 4) heating and maintaining at a glass transition temperature or higher.

Another embodiment of the method for manufacturing a pharmaceutical formulation of the present invention is a method for manufacturing a lyophilized formulation, which lyophilizes the liquid comprising sodium salt of the compound represented by Formula (II), the method comprising at least the following steps:

step 1) cooling a liquid comprising the compound represented by Formula (II) or its pharmaceutically acceptable salt in a chamber of a lyophilizer to a predetermined cooling temperature,
step 2) introducing ice crystals into the chamber,

step 3) further cooling,
step 4) heating and maintaining at a glass transition temperature or higher, and
step 5) drying.

The method for manufacturing a pharmaceutical formulation of the present invention is a method for manufacturing a lyophilized formulation, which lyophilizes a liquid comprising at least the following components:

a) sodium salt of a compound represented by Formula (II):

[Chemical formula 13]

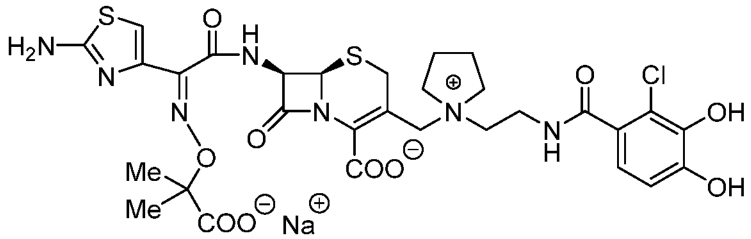

(II)

,

b) one or more selected from the group consisting of alkali metal chloride, alkali earth metal chloride, transition metal chloride and magnesium chloride,
and
c) sugar and/or sugar alcohol,
the method comprising at least the following steps:

step 1) cooling the liquid in a chamber of a lyophilizer to a predetermined cooling temperature, and
step 2) introducing ice crystals into the chamber.

Another embodiment of the method for manufacturing a pharmaceutical preparation of the present invention is a method for manufacturing a lyophilized formulation, which lyophilizes a liquid comprising at least the following components:

a) the sodium salt of the compound represented by Formula (II),
b) one or more selected from the group consisting of alkali metal chloride, alkali earth metal chloride, transition metal chloride and magnesium chloride,
and
c) sugar and/or sugar alcohol,
the method comprising at least the following steps:

step 1) cooling the liquid in a chamber of a lyophilizer to a predetermined cooling temperature,
step 2) introducing ice crystals into the chamber, and
step 3) further cooling.

Another embodiment of the method for manufacturing a pharmaceutical formulation of the present invention is a method for manufacturing a lyophilized formulation, which lyophilizes a liquid comprising at least the following components:

a) the sodium salt of the compound represented by Formula (II),
b) one or more selected from the group consisting of alkali metal chloride, alkali earth metal chloride, transition metal chloride and magnesium chloride,
and
c) sugar and/or sugar alcohol,
the method comprising at least the following steps:

step 1) cooling the liquid in a chamber of a lyophilizer to a predetermined cooling temperature,
step 2) introducing ice crystals into the chamber,
step 3) further cooling, and

step 4) heating and maintaining at a glass transition temperature or higher.

Another embodiment of the method for manufacturing a pharmaceutical formulation of the present invention is a method for manufacturing a lyophilized formulation, which lyophilizes a liquid comprising at least the following components:

a) the sodium salt of the compound represented by Formula (II),
b) one or more selected from the group consisting of alkali metal chloride, alkali earth metal chloride, transition metal chloride and magnesium chloride,
and
c) sugar and/or sugar alcohol,
the method comprising at least the following steps:

step 1) cooling the liquid in a chamber of a lyophilizer to a predetermined cooling temperature,
step 2) introducing ice crystals into the chamber,
step 3) further cooling,
step 4) heating and maintaining at a glass transition temperature or higher, and
step 5) drying.

The method for manufacturing a pharmaceutical formulation of the present invention is a method for manufacturing a lyophilized formulation, which lyophilizes a liquid comprising at least the following components:

a) the sodium salt of the compound represented by Formula (II),
b) sodium chloride,
and
c) sucrose,
the method comprising at least the following steps:

step 1) cooling the liquid in a chamber of a lyophilizer to a predetermined cooling temperature, and
step 2) introducing ice crystals into the chamber.

Another embodiment of the method for manufacturing a pharmaceutical preparation of the present invention is a method for manufacturing a lyophilized formulation, which lyophilizes a liquid comprising at least the following components:

a) the sodium salt of the compound represented by Formula (II),
b) sodium chloride,
and
c) sucrose,
the method comprising at least the following steps:

step 1) cooling the liquid in a chamber of a lyophilizer to a predetermined cooling temperature,
step 2) introducing ice crystals into the chamber, and
step 3) further cooling.

Another embodiment of the method for manufacturing a pharmaceutical formulation of the present invention is a method for manufacturing a lyophilized formulation, which lyophilizes a liquid comprising at least the following components:

a) the sodium salt of the compound represented by Formula (II),
b) sodium chloride,
and
c) sucrose,
the method comprising at least the following steps:

step 1) cooling the liquid in a chamber of a lyophilizer to a predetermined cooling temperature,
step 2) introducing ice crystals into the chamber,
step 3) further cooling, and

step 4) heating and maintaining at a glass transition temperature or higher.

Another embodiment of the method for manufacturing a pharmaceutical formulation of the present invention is a method for manufacturing a lyophilized formulation, which lyophilizes a liquid comprising at least the following components:

a) the sodium salt of the compound represented by Formula (II),
b) sodium chloride,
and
c) sucrose,
the method comprising at least the following steps:

step 1) cooling the liquid in a chamber of a lyophilizer to a predetermined cooling temperature,
step 2) introducing ice crystals into the chamber,
step 3) further cooling,
step 4) heating and maintaining at a glass transition temperature or higher, and
step 5) drying.

Hereinafter, a temperature in the step of the manufacturing method of the present invention indicates a shelf temperature in the lyophilizer. This shelf temperature is approximately equal to a product temperature of the vial in the lyophilizer.

The cooling temperature in the step 1) of the manufacturing method of the present invention is usually -30°C to -5°C, preferably -25°C to -8°C, and more preferably -22°C to -10°C. When the temperature is lower than this temperature, the ice nucleus becomes small, and the pore size of the lyophilized formulation becomes small. As a result, water is less likely to permeate the lyophilized formulation, and a time taken for the lyophilized formulation to be reconstituted with water may be long. When the temperature is higher than this temperature, the water content becomes high, and the compound represented by Formula (I) or its pharmaceutically acceptable salt may be decomposed.

The time in the step 1) of the manufacturing method of the present invention is usually 0.1 hours to 4 hours, preferably 0.25 hours to 3.5 hours, and more preferably 0.5 hours to 3 hours. When the time in the step 1) is shorter than this time, the product temperature may not be sufficiently lowered. When the time in the step 1) is longer than this time, the cooling time is long, and it may take a long time to complete the lyophilized formulation.

The cooling rate in the step 1) of the manufacturing method of the present invention is usually 0.01°C to 2°C/min, preferably 0.05°C to 1.5°C/min, and more preferably 0.1°C to 1°C/min. When the cooling rate is higher than this cooling rate, the product temperature between vials may vary. When the cooling rate is lower than this rate, the cooling time is long, and it may take a long time to complete the lyophilized formulation.

As the ice crystals in the step 2) of the manufacturing method of the present invention, ice crystals (ice fog) are introduced. The raw material of the ice crystals is one or more selected from the group consisting of nitrogen, water vapor, argon, helium, air, oxygen, carbon dioxide, neon, xenon, krypton, and hydrogen. Preferably, the ice crystals can be formed using nitrogen and water vapor (pure steam) as a raw material. Both nitrogen and water vapor (pure steam) are introduced from a nozzle into the lyophilizer.

The generated ice crystals are rapidly introduced into the lyophilization chamber, thereby causing ice nucleation in all the products in the different drug bottles in the production chamber.

The ice crystals themselves can serve as nucleating agents for formation of ice in a supercooled aqueous solution. In this "ice fog" method of introducing ice crystals, the lyophilizer is filled with ice crystals to produce a gas phase suspension of small ice particles. The ice particles are transferred into glass bottles, and ice nucleation is initiated when the ice particles come into contact with a fluid interface.

In the step of introducing ice crystals in the step 2) of the manufacturing method of the present invention, the cooling temperature is usually -30°C to -5°C, preferably -25°C to -8°C, and more preferably -22°C to -10°C. When the temperature is lower than this temperature, the ice nucleus becomes small, and the pore size of the lyophilized formulation becomes small. As a result, water is less likely to permeate the lyophilized formulation, and a time taken for the lyophilized formulation to be reconstituted with water may be long. When the temperature is higher than this temperature, the water content becomes high, and the compound represented by Formula (I) or its pharmaceutically acceptable salt may be decomposed.

The cooling rate in the step 2) of the manufacturing method of the present invention is usually 0.01°C to 2°C/min, preferably 0.05°C to 1.5°C/min, and more preferably 0.1°C to 1°C/min. When the cooling rate in the step 2) is higher than this cooling rate, the particle size of the ice nucleus is small, and the pore size of the lyophilized formulation is small. As a result, water is less likely to permeate the lyophilized formulation, and a time taken for the lyophilized formulation to be reconstituted with water may be long. When the cooling rate in the step 2) is slower than this rate, the cooling time

is long, and it may take a long time to complete the lyophilized formulation.

The cooling temperature in the step 3) of the manufacturing method of the present invention may be any temperature at which the aqueous solution can be frozen. The temperature is usually -80°C to -30°C, preferably -70°C to -35°C, and more preferably -60°C to -40°C.

The time in the step 3) of the manufacturing method of the present invention may be any time as long as the aqueous solution can be frozen, but is usually 2 hours to 8 hours, preferably 2.5 hours to 7.5 hours, and more preferably 3 hours to 7 hours.

The cooling rate in the step 3) of the manufacturing method of the present invention is usually 0.01°C to 2°C/min, preferably 0.05°C to 1.5°C/min, and more preferably 0.1°C to 1.0°C/min.

When sufficient crystallization of a solute or desired polymorph cannot be obtained by a normal lyophilizing process, in order to promote crystallization in the frozen solution, a method in which the product temperature of the frozen solution is once increased to and maintained at a maximum concentrated phase glass transition temperature (Tg') or higher before start of pressure reduction, that is, an "annealing treatment" is effective. The annealing treatment promotes crystallization of the solute, enlarges ice crystals by Ostwald ripening, and increases a water vapor channel that is a rate limiting of the primary drying. As a result, a time required for lyophilizing is shortened, and energy consumption is suppressed. In a case where the step of heating and maintaining at the glass transition temperature or higher in the step 4) of the manufacturing method of the present invention, that is, the "annealing treatment" is performed, sodium sulfate in the formulation of the present invention can be crystallized.

The cooling temperature in the step 4) of the manufacturing method of the present invention may be higher than the cooling temperature in the step 3), and is usually -40°C to -10°C, preferably -35°C to -15°C, and more preferably -30°C to -20°C.

The time in the step 4) of the manufacturing method of the present invention is usually 0.1 hours to 4 hours, preferably 0.25 hours to 3.5 hours, and more preferably 0.5 hours to 3 hours.

The cooling rate in the step 4) of the manufacturing method of the present invention is usually 0.01°C to 2°C/min, preferably 0.05°C to 1.5°C/min, and more preferably 0.1°C to 1°C/min.

As described above, the drying step of the step 5) of the manufacturing method of the present invention is divided into the primary drying involving sublimating the frozen sample under vacuum, and the secondary drying involving heating the sample to a temperature that does not affect the sample, thereby removing water (bound water) bound between molecules. The temperature of the primary drying is usually - 30°C to -1°C, preferably -25°C to -2.5°C, and more preferably -20°C to -5°C.

The time for primary drying of the manufacturing method of the present invention is usually 70 to 100 hours, preferably 75 to 95 hours, and more preferably 80 to 90 hours although it depends on the size of the lyophilizer.

The vacuum pressure for primary drying of the manufacturing method of the present invention is usually 1 to 50 Pa, preferably 2.5 to 40 Pa, and more preferably 5 to 20 Pa.

The temperature for secondary drying of the manufacturing method of the present invention is usually 30°C to 80°C, preferably 35°C to 75°C, and more preferably 40°C to 70°C.

The time for secondary drying of the manufacturing method of the present invention usually 1 hour to 15 hours, preferably 2.5 to 12 hours, and more preferably 5 hours to 10 hours although it depends on the size of the lyophilizer.

The vacuum pressure for secondary drying of the manufacturing method of the present invention is usually 1 to 50 Pa, preferably 2.5 to 40 Pa, and more preferably 5 to 20 Pa.

In the manufacturing method of the present invention, a step of re-cooling may be included between the step 4) and the step 5). The temperature for re-cooling is usually -80°C to -10°C, preferably -70°C to -20°C, and more preferably -60°C to -30°C.

The time in the step of re-cooling is usually 0.25 hours to 3 hours, preferably 0.5 hours to 2.5 hours, and more preferably 0.75 hours to 2 hours.

In the manufacturing method of the present invention, the cooling temperature, the time of the step, and the cooling rate in the case of combining the steps 1) and 2) are usually such that the cooling temperature of the step 1) is -30°C to -5°C, the time of the step 1) is 0.1 hours to 4 hours, the cooling rate of the step 1) is 0.01°C to 2°C/min, the cooling temperature of the step 2) is -30°C to -5°C, and the cooling rate of the step 2) is 0.01°C to 2°C/min. Preferably, the cooling temperature of the step 1) is -25°C to -8°C, the time of the step 1) is 0.25 hours to 3.5 hours, the cooling rate of the step 1) is 0.05°C to 1.5°C/min, the cooling temperature of the step 2) is -25°C to -8°C, and the cooling rate of the step 2) is 0.05°C to 1.5°C/min. More preferably the cooling temperature of the step 1) is -22°C to -10°C, the time of the step 1) is 0.5 hours to 3 hours, the cooling rate of the step 1) is -0.1°C to 1°C/min, the cooling temperature of the step 2) is -22°C to 10°C, and the cooling rate of the step 2) is 0.1°C to 1°C/min. Furthermore, in the step 2), ice crystals are introduced into the lyophilizer.

In the manufacturing method of the present invention, the cooling temperature, the time of the step, and the cooling rate in the case of combining the steps 1), 2) and 3) are usually such that the cooling temperature of the step 1) is - 30°C to -5°C, the time of the step 1) is 0.1 hours to 4 hours, the cooling rate of the step 1) is 0.01°C to 2°C/min,

the cooling temperature of the step 2) is -30°C to -5°C, the cooling rate of the step 2) is 0.01°C to 2°C/min, the cooling temperature of the step 3) is -80°C to -30°C, the time of the step 3) is 2 hours to 8 hours, and the cooling rate of the step 3) is 0.01°C to 2 °C/min. Preferably, the cooling temperature of the step 1) is -25°C to -8°C, the time of the step 1) is 0.25 hours to 3.5 hours, the cooling rate of the step 1) is 0.05°C to 1.5°C/min, the cooling temperature of the step 2) is - 25°C to -8°C, the cooling rate of the step 2) is 0.05°C to 1.5°C/min, the cooling temperature of the step 3) is -70°C to -35°C, the time of the step 3) is 2.5 hours to 7.5 hours, and the cooling rate of the step 3) is 0.05°C to 1.5°C/min. More preferably, the cooling temperature of the step 1) is -22°C to -10°C, the time of the step 1) is 0.5 hours to 3 hours, the cooling rate of the step 1) is 0.1°C to 1°C/min, the cooling temperature of the step 2) is -22°C to -10°C, the cooling rate of the step 2) is 0.1°C to 1°C/min, the cooling temperature of the step 3) is -60°C to -40°C, the time of the step 3) is 3 hours to 7 hours, and the cooling rate of the step 3) is 0.1°C to 1.0 °C/min. Furthermore, in the step 2), ice crystals are introduced into the lyophilizer.

In the manufacturing method of the present invention, the cooling temperature, the time of the step, and the cooling rate in the case of combining the steps 1), 2), 3), and 4) are usually such that the cooling temperature of the step 1) is - 30°C to -5°C, the time of the step 1) is 0.1 hours to 4 hours, the cooling rate of the step 1) is 0.01°C to 2°C/min, the cooling temperature of the step 2) is -30°C to -5°C, the cooling rate of the step 2) is 0.01°C to 2°C/min, the cooling temperature of the step 3) is -80°C to -30°C, the time of the step 3) is 2 hours to 8 hours, the cooling rate of the step 3) is 0.01°C to 2°C/min, the cooling temperature of the step 4) is -40°C to - 10°C, the time of the step 4) is 0.1 hours to 4 hours, and the cooling rate of the step 4) is 0.01°C to 2°C/min. Preferably, the cooling temperature of the step 1) is -25°C to - 8°C, the time of the step 1) is 0.25 hours to 3.5 hours, the cooling rate of the step 1) is 0.05°C to 1.5°C/min, the cooling temperature of the step 2) is -25°C to -8°C, the cooling rate of the step 2) is 0.05°C to 1.5°C/min, the cooling temperature of the step 3) is -70°C to -35°C, the time of the step 3) is 2.5 hours to 7.5 hours, the cooling rate of the step 3) is 0.05°C to 1.5°C/min, the cooling temperature of the step 4) is -35°C to -15°C, the time of the step 4) is 0.25 hours to 3.5 hours, and the cooling rate of the step 4) is 0.05°C to 1.5°C/min. More preferably, the cooling temperature of the step 1) is -22°C to -10°C, the time of the step 1) is 0.5 hours to 3 hours, the cooling rate of the step 1) is 0.1°C to 1°C/min, the cooling temperature of the step 2) is -22°C to - 10°C, the cooling rate of the step 2) is 0.1°C to 1°C/min, the cooling temperature of the step 3) is -60°C to -40°C, the time of the step 3) is 3 hours to 7 hours, the cooling rate of the step 3) is 0.1°C to 1.0°C/min, the cooling temperature of the step 4) is - 30°C to -20°C, the time of the step 4) is 0.5 hours to 3 hours, and the cooling rate of the step 4) is 0.1°C to 1.0°C/min. Furthermore, in the step 2), ice crystals are introduced into the lyophilizer.

In the manufacturing method of the present invention, the cooling temperature, the time of the step, and the cooling rate in the case of combining the steps 1), 2), 3), 4), and 5) are usually such that the cooling temperature of the step 1) is -30°C to -5°C, the time of the step 1) is 0.1 hours to 4 hours, the cooling rate of the step 1) is 0.01°C to 2°C/min, the cooling temperature of the step 2) is -30°C to -5°C, the cooling rate of the step 2) is 0.01°C to 2°C/min, the cooling temperature of the step 3) is -80°C to -30°C, the time of the step 3) is 2 hours to 8 hours, the cooling rate of the step 3) is 0.01°C to 2°C/min, the cooling temperature of the step 4) is -40°C to - 10°C, the time of the step 4) is 0.1 hours to 4 hours, the cooling rate of the step 4) is 0.01°C to 2°C/min, the temperature of the primary drying of the step 5) is -30°C to - 1°C, the time of the primary drying is 70 hours to 100 hours, the vacuum pressure of the primary drying is 1 to 50 Pa, the temperature of the secondary drying is 30°C to 80°C, the time of the secondary drying is 1 to 15 hours, and the vacuum pressure of the secondary drying is 1 to 50 Pa. Preferably, the cooling temperature of the step 1) is -25°C to -8°C, the time of the step 1) is 0.25 hours to 3.5 hours, the cooling rate of the step 1) is 0.05°C to 1.5°C/min, the cooling temperature of the step 2) is -25°C to - 8°C, the cooling rate of the step 2) is 0.05°C to 1.5°C/min, the cooling temperature of the step 3) is -70°C to -35°C, the time of the step 3) is 2.5 hours to 7.5 hours, the cooling rate of the step 3) is 0.05°C to 1.5°C/min, the cooling temperature of the step 4) is -35°C to -15°C, the cooling time of the step 4) is 0.25 hours to 3.5 hours, the cooling rate of the step 4) is 0.05°C to 1.5°C/min, the temperature of the primary drying of the step 5) is -25°C to -2.5°C, the time of the primary drying is 75 hours to 95 hours, the vacuum pressure of the primary drying is 2.5 to 40 Pa, the temperature of the secondary drying is 35°C to 75°C, the time of the secondary drying is 2.5 to 12 hours, and the vacuum pressure of the secondary drying is 2.5 to 40 Pa. More preferably, the cooling temperature of the step 1) is -22°C to -10°C, the time of the step 1) is 0.5 hours to 3 hours, the cooling rate of the step 1) is 0.1°C to 1°C/min, the cooling temperature of the step 2) is -22°C to -10°C, the cooling rate of the step 2) is 0.1°C to 1°C/min, the cooling temperature of the step 3) is -60°C to -40°C, the time of the step 3) is 3 hours to 7 hours, the cooling rate of the step 3) is 0.1°C to 1.0°C/min, the cooling temperature of the step 4) is -30°C to -20°C, the time of the step 4) is 0.5 hours to 3 hours, the cooling rate of the step 4) is 0.1°C to 1.0°C/min, the temperature of the primary drying of the step 5) is -20°C to -5°C, the time of the primary drying is 80 hours to 90 hours, the vacuum pressure of the primary drying is 5 to 20 Pa, the temperature of the secondary drying is 40°C to 70°C, the time of the secondary drying is 5 to 10 hours, and the vacuum pressure of the secondary drying is 5 to 20 Pa. Furthermore, in the step 2), ice crystals are introduced into the lyophilizer.

As a method for manufacturing a liquid before lyophilizing, the liquid before lyophilizing can be manufactured by a manufacturing method comprising the following steps:

1) a step pf adjusting a pH of a liquid comprising the compound represented by Formula (I):

[Chemical formula 14]

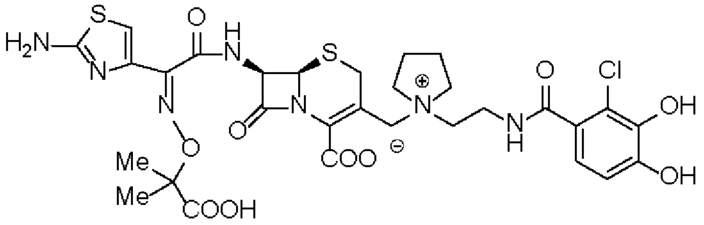 (I)

or its pharmaceutically acceptable salt to 5 to 6 with an alkaline substance, and
2) a step of mixing the liquid manufactured in the step 1), a component b): one or more selected from the group consisting of alkali metal chloride, alkali earth metal chloride, transition metal chloride and magnesium chloride and a component c): sugar and/or sugar alcohol. The component b) is preferably sodium chloride, and the component c) is preferably sucrose.

As the method for manufacturing a liquid before lyophilizing, more preferably, the liquid before lyophilizing can be manufactured by a manufacturing method comprising at least the following steps:

1) a step of adjusting a pH of the liquid comprising the compound represented by Formula (I) or its pharmaceutically acceptable salt to 5.5 to 6 with an alkaline substance, and
2) a step of mixing the liquid manufactured in the step 1), the component b) and the component c),

wherein the components b) and c) have the same meaning as described above.
As the method for manufacturing a liquid before lyophilizing, still more preferably, the liquid before lyophilizing can be manufactured by a manufacturing method comprising at least the following steps:

1) a step of adjusting a pH of a suspension of the compound represented by Formula (I) or its pharmaceutically acceptable salt to 5 to 6 with sodium hydroxide, and
2) a step of mixing the liquid manufactured in the step 1), the component b) and the component c),

wherein the components b) and c) have the same meaning as described above.
Still even more preferably, the liquid before lyophilizing can be manufactured by a manufacturing method comprising at least the following steps:

1) a step of adjusting a pH of a suspension of the compound represented by Formula (I) or its pharmaceutically acceptable salt to 5.5 to 6 with sodium hydroxide, and
2) a step of mixing the liquid manufactured in the step 1), the component b) and the component c),

wherein the components b) and c) have the same meaning as described above.
More preferably, the liquid before lyophilizing can be manufactured by a manufacturing method comprising at least the following steps:

1) a step of adjusting a pH of a suspension of p-toluenesulfonic acid salt and sulfuric acid salt of the compound represented by Formula (I) to 5.5 to 6 with sodium hydroxide, and
2) a step of mixing the liquid manufactured in the step 1), the component b) and the component c), wherein the components b) and c) have the same meaning as described above.

In the case of manufacturing the pharmaceutical formulation of the present invention, the formulation is manufactured as follows: 1) acidic slurry solution is prepared by adding the compound represented by Formula (I) or its pharmaceutically acceptable salt, preferably, p-toluenesulfonic acid salt and sulfuric acid salt of the compound represented by Formula (I) in injectable water, 2) sodium hydroxide aqueous solution is added in the slurry solution of said 1), and the pH of the solution is adjusted to 5.5 to 6, and the additive is added to stabilize the slurry solution of said 1), 3) injectable water is added additionally and the concentration of the solution is adjusted to 10 w/w%, the pH of the solution is adjusted to 5 to 6, and the solution is aseptically filtered and formulation solution is completed, 4) a given

amount of said 3) formulation solution is dispensed in a vial or ampule and the like and the formulation is manufactured by lyophilization. The manufacture of the pharmaceutical formulation of the present invention is preferably performed under the sealing condition.

Sterile filtration can be performed by a sterile filtration filter.

As the vial bottle, a clear glass vial bottle having a volume of about 5 to 20 mL can be used. For example, a BVK type vial bottle can be used.

The lyophilized pharmaceutical formulation of the present invention is administered after adding a solution such as water for injection, normal saline solution or glucose solution at the time of use to dissolve. The pharmaceutical formulation of the present invention exhibits a strong antibacterial spectrum against Gram-positive bacteria and Gram-negative bacteria, exhibits antibacterial activity especially against β-lactamase-producing Gram-negative bacteria, and does not exhibit cross-resistance with existing cephem drugs and carbapenems.

The pharmaceutical formulation of the present invention comprising the compound represented by Formula (I) or its pharmaceutically acceptable salt, and the sodium salt of the compound represented by Formula (II) is administered as an injection.

The specific surface area of the pharmaceutical formulation of the present invention can be controlled by the manufacturing method of the present invention. The specific surface area can be controlled by a cooling temperature and a cooling rate at the time of introducing the ice crystals. When the cooling temperature at the time of introducing the ice crystals is high, the particle size of the ice nucleus at the time of freezing becomes large. When the cooling temperature at the time of introducing the ice crystals is low, the particle size of the ice nucleus at the time of freezing becomes small. When the cooling rate is high, the particle size of the ice nucleus at the time of freezing is large. When the cooling rate is low, the particle size of the ice nucleus at the time of freezing is large.

Through the primary drying, the water vapor is sublimated from the ice nucleus, and the trace of ice nucleus of the lyophilized formulation becomes a pore. Therefore, when the particle size of the ice nucleus at the time of freezing increases, the pore which is the trace of ice nucleus of the lyophilized formulation increases. When the particle size of the ice nucleus at the time of freezing decreases, the pore which is the trace of the ice nucleus of the lyophilized formulation decreases.

When the size of the pore of the lyophilized formulation increases, the specific surface area thereof decreases, but the permeation rate of water into the lyophilized formulation becomes faster and the dissolution time, that is, the reconstitution time of the lyophilized formulation becomes shorter. On the other hand, when the size of the pore of the lyophilized formulation decreases, the specific surface area thereof increases, but the permeation rate of water into the lyophilized formulation becomes slower and the dissolution time, that is, the reconstitution time of the lyophilized formulation becomes longer.

The term "reconstitution time" means a time required for the lyophilized molecules to be dissolved and/or suspended in liquid form. For example, the reconstitution time includes, but is not limited to, a time required for a lyophilized formulation comprising the compound represented by Formula (I) or its pharmaceutically acceptable salt to be suspended in water or a buffer after lyophilization.

The reconstitution time of the pharmaceutical formulation of the present invention is usually 30 seconds or less, preferably 28 seconds or less, and more preferably 25 seconds or less.

When the size of the pore of the lyophilized formulation increases, that is, when the specific surface area thereof decreases, the water content in the lyophilized formulation increases. In such a case, a compound that is easily decomposed with water or its pharmaceutically acceptable salt, such as the compound represented by Formula (I) or its pharmaceutically acceptable salt, is decomposed, and there is a possibility that a degradant is generated. On the other hand, when the size of the pore of the lyophilized formulation decreases, that is, when the specific surface area thereof increases, the water content in the lyophilized formulation decreases. In such a case, a compound that is easily decomposed with water or its pharmaceutically acceptable salt, such as the compound represented by Formula (I) or its pharmaceutically acceptable salt, is hardly decomposed, and there is little possibility that a degradant is generated.

The water content of the pharmaceutical formulation of the present invention is only required to be 0.5% or less. When the water content is larger than this water content, the amount of the degradant may increase to the standard value or more after storage for 4 years under refrigeration (2 to 8°C).

In order to optimize the reconstitution time and the water content, the specific surface area of the pharmaceutical formulation of the present invention is 0.6 $m^2/g$ to 1.1 $m^2/g$, preferably 0.625 $m^2/g$ to 1.1 $m^2/g$, and more preferably 0.65 $m^2/g$ to 1.1 $m^2/g$. When the specific surface area is larger than this specific surface area, the reconstitution time may be long. When the specific surface area is smaller than this specific surface area, the water content may be large. A lyophilized formulation having the above-described specific surface area may be manufactured by the manufacturing method of the present invention, but can be manufactured by a method other than the manufacturing method of the present invention. Even when the formulation is not manufactured by the manufacturing method of the present invention, the optimum reconstitution time and water content can be achieved as long as the lyophilized formulation has the above-described specific surface area.

When lyophilization is performed using a large number of vials, the specific surface area of the lyophilized formulation may vary for each vial. However, in the case of the manufacturing method of the present invention, the variation in the specific surface area of the lyophilized formulation per vial is small. The specific surface area is usually 0.3 $m^2/g$ or less, preferably 0.25 $m^2/g$ or less, and more preferably 0.2 $m^2/g$ or less.

When the water content in the lyophilized formulation comprising the compound represented by Formula (I) is higher than 0.5%, there is a possibility that the amount of the degradant increases after storage for 4 years under refrigeration (2 to 8°C). However, the degradant is mainly a compound represented by Formula (III):

[Chemical formula 15]

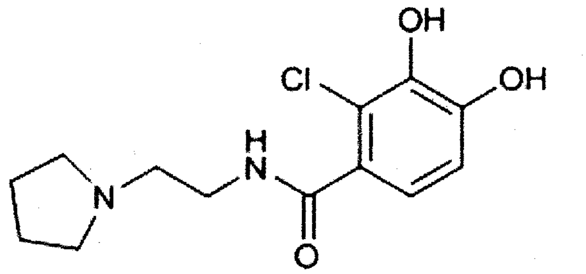

(III)

An increased amount of the compound represented by Formula (III) is preferably low in consideration of toxicity. The tolerance of content of the compound in the lyophilized formulation after storage for 4 years under refrigeration (2 to 8°C) is 1.30% peak area ratio (liquid chromatography) or less.

When the lyophilized formulation is a lyophilized formulation comprising a compound represented by Formula (I):

[Chemical formula 16]

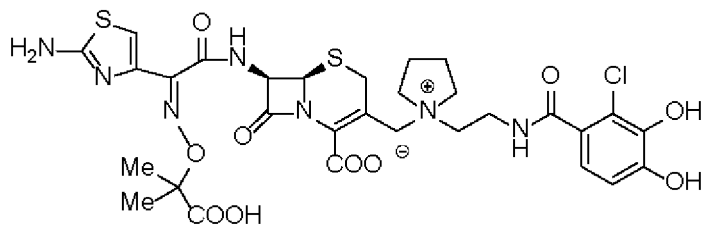

(I)

or its pharmaceutically acceptable salt, and the specific surface area of the lyophilized formulation is 0.6 to 1.1 $m^2/g$, the reconstitution time is 30 seconds or less, and the water content is 0.5% or less. Preferably, when the specific surface area of the lyophilized formulation is 0.625 to 1.1 $m^2/g$, the reconstitution time is 28 seconds or less, and the water content is 0.5% or less. More preferably, when the specific surface area of the lyophilized formulation is 0.65 to 1.1 $m^2/g$, the reconstitution time is 25 seconds or less, and the water content is 0.5% or less.

When the lyophilized formulation is a lyophilized formulation comprising at least the following components:

a) the compound represented by Formula (I) or its pharmaceutically acceptable salt,
b) one or more selected from the group consisting of alkali metal chloride, alkali earth metal chloride, transition metal chloride and magnesium chloride,
and
c) sugar and/or sugar alcohol,

and the specific surface area of the lyophilized formulation is 0.6 to 1.1 $m^2/g$, the reconstitution time is 30 seconds or less, and the water content is 0.5% or less. Preferably, when the specific surface area of the lyophilized formulation is 0.625 to 1.1 $m^2/g$, the reconstitution time is 28 seconds or less, and the water content is 0.5% or less. More preferably, when the specific surface area of the lyophilized formulation is 0.65 to 1.1 $m^2/g$, the reconstitution time is 25 seconds or less, and the water content is 0.5% or less.

When the lyophilized formulation is a lyophilized formulation comprising at least the following components:

a) the compound represented by Formula (I) or its pharmaceutically acceptable salt,
b) sodium chloride,
and
c) sucrose,

and the specific surface area of the lyophilized formulation is 0.6 to 1.1 $m^2/g$, the reconstitution time is 30 seconds or less, and the water content is 0.5% or less. Preferably, when the specific surface area of the lyophilized formulation is 0.625 to 1.1 $m^2/g$, the reconstitution time is 28 seconds or less, and the water content is 0.5% or less. More preferably, when the specific surface area of the lyophilized formulation is 0.65 to 1.1 $m^2/g$, the reconstitution time is 25 seconds or less, and the water content is 0.5% or less.

When the lyophilized formulation is a lyophilized formulation comprising sodium salt of a compound represented by Formula (II):

[Chemical formula 17]

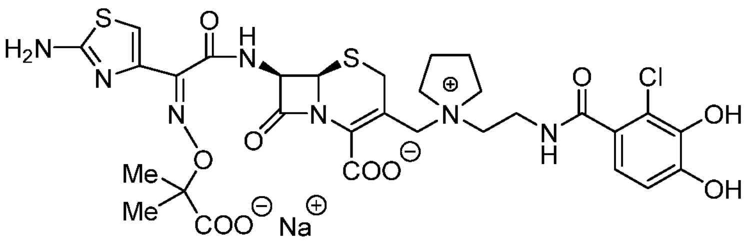

(II)

,

and the specific surface area of the lyophilized formulation is 0.6 to 1.1 $m^2/g$, the reconstitution time is 30 seconds or less, and the water content is 0.5% or less. Preferably, when the specific surface area of the lyophilized formulation is 0.625 to 1.1 $m^2/g$, the reconstitution time is 28 seconds or less, and the water content is 0.5% or less. More preferably, when the specific surface area of the lyophilized formulation is 0.65 to 1.1 $m^2/g$, the reconstitution time is 25 seconds or less, and the water content is 0.5% or less.

When the lyophilized formulation is a lyophilized formulation comprising at least the following components:

a) the sodium salt of the compound represented by Formula (II),
b) one or more selected from the group consisting of alkali metal chloride, alkali earth metal chloride, transition metal chloride and magnesium chloride,
and
c) sugar and/or sugar alcohol,

and the specific surface area of the lyophilized formulation is 0.6 to 1.1 $m^2/g$, the reconstitution time is 30 seconds or less, and the water content is 0.5% or less. Preferably, when the specific surface area of the lyophilized formulation is 0.625 to 1.1 $m^2/g$, the reconstitution time is 28 seconds or less, and the water content is 0.5% or less. More preferably, when the specific surface area of the lyophilized formulation is 0.65 to 1.1 $m^2/g$, the reconstitution time is 25 seconds or less, and the water content is 0.5% or less.

When the lyophilized formulation is a lyophilized formulation comprising at least the following components:

a) the sodium salt of the compound represented by Formula (II),
b) sodium chloride,
and
c) sucrose,

and the specific surface area of the lyophilized formulation is 0.6 to 1.1 $m^2/g$, the reconstitution time is 30 seconds or less, and the water content is 0.5% or less. Preferably, when the specific surface area of the lyophilized formulation is 0.625 to 1.1 $m^2/g$, the reconstitution time is 28 seconds or less, and the water content is 0.5% or less. More preferably, when the specific surface area of the lyophilized formulation is 0.65 to 1.1 $m^2/g$, the reconstitution time is 25 seconds or less, and the water content is 0.5% or less.

[EXAMPLES]

The present invention will be explained in more detail below by way of Examples, and Comparative Examples,

but these do not limit the present invention.

1. The method for manufacturing the compound represented by Formula (I) or its pharmaceutically acceptable salt (1.3 molar equivalent of tosic acid salt/0.4 molar equivalent of sulfuric acid salt)

For example, the compound represented by Formula (I) or its pharmaceutically acceptable salt can be synthesized with reference to International Publication WO 2016/035845A and International Publication WO 2016/035846A.

<Preparation of seed crystals A of 2 molar equivalent of p-toluenesulfonic acid salt of the compound represented by Formula (I)>

The compound represented by Formula (I) (100 mg) was dissolved in 1.0 mol/L p-toluenesulfonic acid aqueous solution (2 mL) at room temperature using an ultrasonic, and the resulting solution was left to stand at 4°C for 4 days. The precipitate was filtered to yield seed crystals A (73 mg). The seed crystals A were observed with a microscope, and confirmed to be needle-like crystals.

<Preparation of hydrate crystals (hereinafter, referred to as "crystal form I") of a mixed acid salt of 1.3 molar equivalent of p-toluenesulfonic acid and 0.35 molar equivalent of sulfuric acid of the compound represented by Formula (I)>

Step 1: Preparation of seed crystals C

The seed crystals A (50 mg) were dissolved in 6 mol/L $H_2SO_4$ (3 mL) on an ultrasonic water bath at room temperature, and the solution was left to stand at 4°C for 2 days. The precipitated crystalline solid was filtered and washed with ice chilled water to yield seed crystals C (23 mg).

Step 2: Synthesis of the compound 5

[Chemical formula 18]

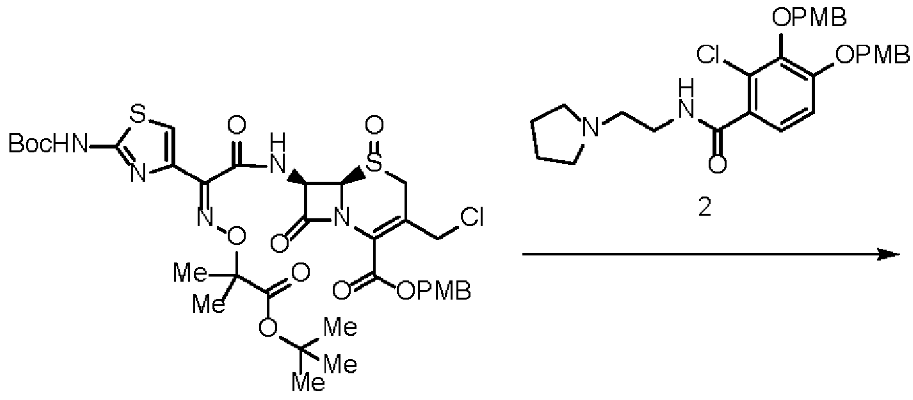

2

1

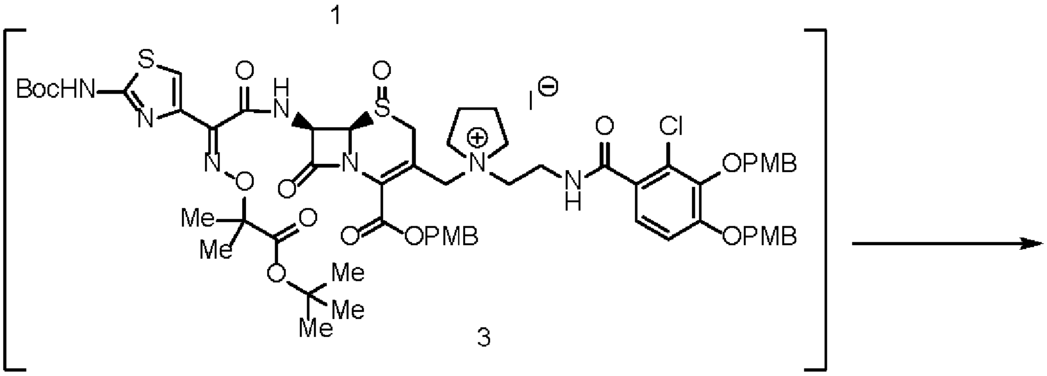

3

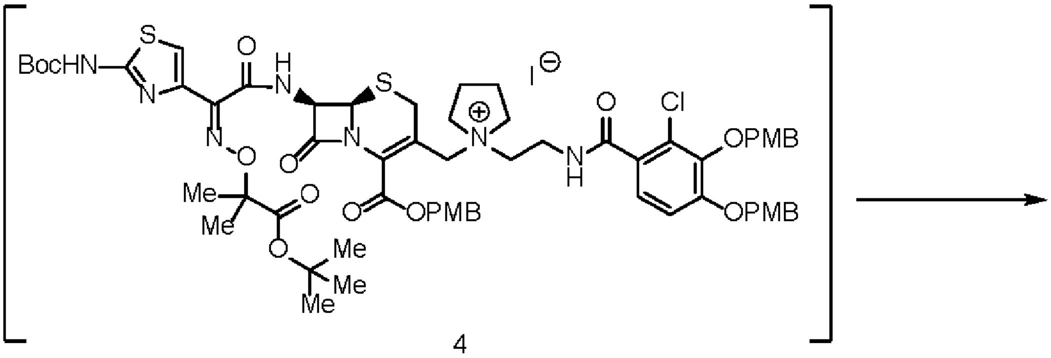

4

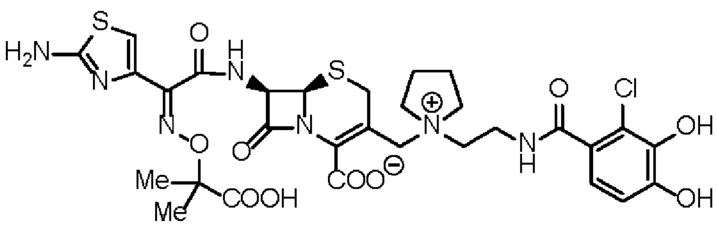

5

Abbreviations used herein are defined as follows.

Boc: t-butoxycarbonyl

PMB: p-methoxybenzyl

Under a nitrogen atmosphere, the compound 1 (18.0 kg, 22.6 mol) was dissolved in N,N-dimethylacetamide (41 L), and the solution was cooled to 0°C. Sodium iodide (6.8 kg, 45.2 mol), the compound 2 (13.1 kg, 24.9 mol), and N,N-dimethylacetamide (4 L) were added to the solution, and the mixed solution was stirred at 0°C for 6 hours. The solution was warmed to 7°C, and stirred for 16 hours. The solution was cooled to 0°C and sodium iodide (5.1 kg, 33.9

mol) was added to the solution, and then acetyl chloride (8.9 kg, 113.0 mol) was added dropwise to the solution over 90 minutes at 0°C, the solution was stirred at 0°C for 5 hours.

Anisole (36 L) was added to the reaction solution, this solution was added to a mixed solution of methyl ethyl ketone and sodium bisulfite aqueous solution, and the resulting solution was subjected to extraction. The organic layer was washed with a mixed solution of sulfuric acid and brine twice. Anisole (90 L) was added to the washed product, the resulting solution was cooled to 15°C. Then, 75% sulfuric acid (36.0 kg) was added to the solution, and the resulting solution was stirred at 28°C for 2 hours. After adding water (90 L) and ethyl acetate (36 L), the resulting solution was subjected to extraction. The obtained aqueous layer was washed with ethyl acetate twice, and then purified by reverse phase column chromatography (acetonitrile-sulfuric acid aqueous solution) using a chromatographic separation small particle size synthetic adsorbent (Diaion™ HP20SS). After adding an aqueous solution of 75% sulfuric acid (33.4 kg) and p-toluenesulfonic acid monohydrate (16.7 kg) to the obtained eluate, an appropriate amount of the seed crystal C was added to the solution to precipitate a solid. It was cooled to 5°C and stirred at 5°C for 10 hours, and the precipitated crystalline solid was filtered. The crystalline solid was washed with water cooled to 5°C, and then dried under reduced pressure to yield a crystal form I of the compound 5, that is, p-toluenesulfonic acid salt and sulfuric acid salt of the compound represented by Formula (I) (1.3 molar equivalent of tosic acid salt/0.35 molar equivalent of sulfuric acid salt) (12.7 kg, content conversion yield: 49%).

The contents of p-toluenesulfonic acid and sulfuric acid in the crystal form I were determined by the following method.

(p-Toluenesulfonic acid content measuring method)

Step 1: Preparation of a sample solution

About 40 mg of the sample was weighed precisely, and dissolved in a sample dilution solvent to prepare exactly 25 mL solution. To 2 mL of this solution weighed precisely was added a sample dilution solvent to prepare exactly 20 mL solution.

Step 2: Preparation of a standard solution

About 25 mg of a standard preparation of sodium p-toluene sulfonate equilibrated humidity under the condition of 25°C/60% RH was weighed precisely, and dissolved in a sample dilution solvent to prepare exactly 100 mL solution. To 5 mL of this solution weighed precisely was added a sample dilution solvent to prepare exactly 50 mL solution.

As the above sample dilution solvent, a mixture of 5 mmol/L phosphate buffer/liquid chromatography acetonitrile (9 : 1) was used. Herein, a mixture of water: 0.05 mol/L sodium dihydrogen phosphate test solution: 0.05 mol/L disodium hydrogen phosphate test solution = 18 : 1 : 1 (pH is about 7.1 ) was used as the phosphate buffer.

Step 3: Measurement and determination

The peak area of p-toluenesulfonic acid was determined in an automatic integration method by measuring the above sample solution and the standard solution under the following test condition by liquid chromatography. The term "on an anhydrous basis" refers to a value calculated with a value obtained by subtracting the water content from the total amount as 100%. The gradient program of the mobile phase is shown in Table 1.

(Test condition)

Column: Unison UK-C18, $\phi$4.6 × 150 mm, 3 $\mu$m, manufactured by Imtakt
Column temperature: constant temperature near 35°C
Flow rate: 1.0 mL per minute (a retention time of p-toluenesulfonic acid: about 7 minutes)
Detector: ultraviolet absorption spectrophotometer (wavelength: 218 nm)
Mobile phase A: 0.1% trifluoroacetic acid solution
Mobile phase B: liquid chromatography acetonitrile

[Table 1]

| Gradient program | | |
|---|---|---|
| Time after injection (min) | Mobile phase A (vol%) | Mobile phase B (vol%) |
| 0 - 7 | 95 | 5 |
| 7 - 7.01 | 95 → 60 | 5 → 40 |
| 7.01 - 15 | 60 | 40 |
| 15 - 15.01 | 60 → 95 | 40 → 5 |
| 15.01 - 25 | 95 | 5 |

The content of p-toluenesulfonic acid in the sample was determined using the following Formula.

$$\text{The amount of p-toluenesulfonic acid (\%)} = \frac{M_S}{M_T} \times \frac{P}{100} \times \frac{100}{100 - W_T} \times \frac{A_T}{A_S} \times \frac{172.20}{194.18} \times \frac{1}{4} \times 100$$

$M_S$: weighed amount of a standard preparation of sodium p-toluene sulfonate (mg)
$M_T$: weighed amount of a sample (mg)
P: purity of a standard preparation of sodium p-toluene sulfonate (%)
$W_T$: moisture of a sample (%)
$A_T$: peak area of p-toluenesulfonic acid obtained from the sample solution
$A_S$: peak area of p-toluenesulfonic acid obtained from the standard solution
172.20: molecular weight of p-toluenesulfonic acid
194.18: molecular weight of sodium p-toluene sulfonate

$\frac{1}{4}$ : dilution rate

(Sulfuric acid content measuring method)

Step 1: Preparation of a standard solution

About 50 mg of sodium sulfate anhydrous was weighed precisely, and dissolved in a mobile phase to prepare exactly 25 mL solution. To 2 mL of this solution weighed precisely was added a mobile phase to prepare exactly 50 mL solution. Furthermore, to 2 mL of this solution weighed precisely was added a mobile phase to prepare exactly 20 mL solution.

Step 2: Preparation of a sample solution

About 30 mg of a sample was weighed precisely, and dissolved in a mobile phase to prepare exactly 25 mL solution. To 2 mL of this solution weighed precisely was added a mobile phase to prepare exactly 20 mL solution.

Step 3: Measurement and determination

The peak area of sulfate ion was determined in an automatic integration method by measuring the above sample solution and the standard solution under the following test condition by liquid chromatography.

(Test condition)

Column: Shim-pack IC-A3, ϕ4.6 × 150 mm, 5 μm, Shimadzu Corporation
Column temperature: constant temperature near 40°C

Flow rate: 1.2 mL per minute (a retention time of sulfate ion: about 15 minutes)
Detector: electric conductivity detector (non-suppressor system)
Mobile phase: a solution obtained by precisely weighing about 0.67 g of Bis-Tris, about 3.09 g of boric acid, and about 1.11 g of ground p-hydroxybenzoic acid, and dissolving them in water to make the volume exactly 1,000 mL.

The content of sulfuric acid in the sample was determined using the following formula.

$$\text{The amount of sulfuric acid (\%)} = M_S/M_T \times 100/(100 - W_T) \times A_T/A_S \times 98.08/142.04 \times 1/25 \times 100$$

$M_S$: weighed amount of sodium sulfate anhydrous (mg)
$M_T$: weighed amount of a sample (mg)
$W_T$: moisture of a sample (%)
$A_S$: peak area of sulfate ion obtained from the standard solution
$A_T$: peak area of sulfate ion obtained from the sample solution
98.08: molecular weight of sulfuric acid
142.04: molecular weight of sodium sulfate anhydrous
1/25: dilution rate

(Result)

p-Toluenesulfonic acid: 22.2±0.2% (on an anhydrous basis)
Sulfuric acid: 4.3%±0.1% (on an anhydrous basis)

Elemental analysis: (calculated as

$$C_{30}H_{34}N_7ClO_{10}S_2 \cdot 1.32C_7H_8O_3S \cdot 0.45H_2SO_4 \cdot 9.0H_2O)$$

Calculated: C 39.75 (%), H 5.39 (%), N 8.27 (%), C1 2.99 (%), S 10.19 (%), $H_2O$ 13.67 (%)
Measured: C 39.73 (%), H 5.33 (%), N 8.53 (%), C1 3.08 (%), S 10.11 (%), $H_2O$ (KF method) 13.69 (%)
As described above, the obtained crystals are crystal form I which are hydrate crystals of mixed acid salt of 1.3 molar equivalent of p-toluenesulfonic acid and 0.35 molar equivalent of sulfuric acid of the compound represented by Formula (I), and in which about 0.02 molar equivalent of p-toluenesulfonic acid and about 0.1 molar equivalent of sulfuric acid remain. Even in a crystal in which p-toluenesulfonic acid and/or sulfuric acid further remain in the crystal form I, the characteristic powder X-ray diffraction pattern of the crystal form I is not changed. Such a crystal can be stably present substantially as the same crystal as the crystal form I. The crystal form I may include those comprising about 0.01 to 0.1 molar equivalent of p-toluenesulfonic acid and/or about 0.01 to 0.1 molar equivalent of sulfuric acid remaining therein. The remaining acid may be in the form of being adhered to a crystal or the form of being incorporated into a crystal.
A preferable content of p-toluenesulfonic acid in the crystal form I is about 20.2±0.2 to 23.2±0.2% (on an anhydrous basis), and a preferable content of sulfuric acid is about 3.5±0.1 to 5.0±0.1% (on an anhydrous basis). A more preferable content of p-toluenesulfonic acid in the crystal form I is about 21.5±0.2 to 22.3±0.2% (on an anhydrous basis), and a more preferable content of sulfuric acid is about 4.2±0.1 to 4.9±0.1% (on an anhydrous basis). A further preferable content of p-toluenesulfonic acid in the crystal form I is about 21.5 to 22.3% (on an anhydrous basis), and a further preferable content of sulfuric acid is about 4.2 to 4.9% (on an anhydrous basis).
2. Manufacturing method of the liquid comprising sodium salt of the compound represented by Formula (II), sodium p-toluene sulfonate and sodium sulfate
For example, the liquid can be manufactured with reference to International Publication WO 2016/035845A and International Publication WO 2016/035846A.
The sodium salt of the compound represented by Formula (II) (hereinafter, sometimes abbreviated as "sodium salt of Formula (II)"), sodium p-toluene sulfonate (hereinafter, sometimes abbreviated as "sodium tosylate"), sodium sulfate (hereinafter, sometimes abbreviated as "sodium sulfate") were manufactured by the following method. That is, 1,000 mg of the compound represented by Formula (I) or its pharmaceutically acceptable salt, acid addition salt, preferably, crystal form I was weighed in terms of the compound represented by Formula (I), and the crystals were suspended in about 4.5 mL water for injection. The pH of the suspension was adjusted to 5.5 to 6 with 12% sodium hydroxide aqueous solution, and the crystals were dissolved as sodium salt of the compound represented by Formula (II). The

additives for stabilization are added to the solution, injectable water is added additionally, the concentration is adjusted to 10 wt%, the pH is adjusted to 5 to 6, and the solution is aseptically filtered and formulation solution is prepared. A given amount of the formulation solution is dispensed in a vial or ampule and the like, and the preparation is manufactured by lyophilizing the formulation solution. By this method, a liquid comprising 1,000 mg (in terms of the compound represented by Formula (I)) of the sodium salt of the compound represented by Formula (II), 334.5 mg of sodium p-toluene sulfonate, and 84.8 mg of sodium sulfate was manufactured. The active ingredient of the following example exists as sodium salt of the compound represented by Formula (II), but, in the table, the sodium salt of the compound represented by Formula (II) is converted by the compound represented by Formula (I).

3. Lyophilized formulation manufactured by a lyophilizer (laboratory machine) (1) Lyophilizer and other equipment

As the lyophilizer and the wireless product temperature sensor for measuring the product temperature, those listed in Table 2 were used.

Table 2

| Equipment/device name | Model | Manufacturer |
|---|---|---|
| Lyophilizer | Triomaster II A-04 | Kyowa Vacuum Engineering Co., Ltd |
| Lyophilizer | Lyostar II | SP Scientific |
| Wireless product temperature sensor | TEMPRIS Mini LAB | IQ-mobil |

(2) Components and composition of lyophilized formulation

The components and composition (per one vial) of the lyophilized formulation comprising the sodium salt of the compound represented by Formula (II), sodium p-toluene sulfonate, and sodium sulfate are shown in Table 3.

[Table 3]

| | Component amount per vial (mg) |
|---|---|
| Sodium salt of the compound represented by Formula (II) | 1000.0 |
| Sodium p-toluene sulfonate | 334.5 |
| Sodium sulfate | 84.8 |
| Sucrose | 900.0 |
| Sodium chloride | 216.0 |

(3) Formulation solution preparation method

First, 1,000 mg of crystal form I was weighed in terms of the compound represented by Formula (I), and these crystals were added into a vial bottle and suspended in water for injection. The pH of the suspension was adjusted to 5.5 to 6 with 12% sodium hydroxide solution, and the crystals were dissolved as the sodium salt of the compound represented by Formula (II). Then, 900 mg of sucrose (manufactured by Merck) and 216 mg of sodium chloride (manufactured by Merck) were added to this solution. After stirring and dissolution, water for injection was added to the solution. The concentration of the solution was adjusted to 10 wt% in terms of the compound represented by Formula (I) and the pH was adjusted to 5 to 6. Thereafter, the solution was sterile filtered and the formulation solution was prepared. The formulation solution was filtered through a PVDF membrane filter having a pore size of 0.2 $\mu$m. A 14 mL clear glass vial was filled with 10 g of the filtrate, and the vial was semi-stoppered with a rubber stopper and then loaded into a lyophilizer.

(4) Manufacturing conditions of lyophilized formulation

Among the manufacturing conditions of the lyophilized formulation in the lyophilizer (laboratory machine), the cooling conditions are shown in Table 4, and the drying conditions are shown in Table 5. The manufacturing of the lyophilized formulation is performed by 1) a step of cooling a liquid comprising the compound represented by Formula (I) or its pharmaceutically acceptable salt in a chamber of a lyophilizer to a predetermined cooling temperature, 2) a step of introducing ice crystals into the chamber, 3) a step of further cooling, 4) a step of heating and maintaining at the glass

transition temperature or higher, 5) a primary drying step, and 6) a secondary drying step. In Table 4, the "step of cooling to a predetermined cooling temperature and introducing ice crystals" refers to the above steps 1) and 2), the "step of further cooling" refers to the above step 3), and the "step of heating and maintaining at the glass transition temperature or higher" refers to the above step 4). In Table 5, the "primary drying step" refers to the above step 5), and the "secondary drying step" refers to the above step 6). In some examples and reference examples, 4) the step of heating and maintaining at the glass transition temperature or higher is not performed in some cases (this case is represented by "not performed" in Table 4).

[Table 4]

| Formulation Example | Manufacturing scale (number of vials) | Step of cooling to a predetermined cooling temperature and introducing ice crystals | | Step of further cooling | | Step of heating and maintaining at the glass transition temperature or higher | |
|---|---|---|---|---|---|---|---|
| | | Temperature (°C) | Time (hr) | Temperature (°C) | Time (hr) | Temperature (°C) | Time (hr) |
| Example 1 | 38 | -10 | 3 | -47.5 | 4 | Not performed | Not performed |
| Example 2 | 38 | -10 | 3 | -47.5 | 4 | Not performed | Not performed |
| Example 3 | 38 | -12.5 | 3 | -47.5 | 4 | Not performed | Not performed |
| Example 4 | 38 | -12.5 | 3 | -47.5 | 4 | Not performed | Not performed |
| Example 5 | 49 | -15 | 3 | -40 | 4 | Not performed | Not performed |
| Example 6 | 38 | -10 | 3 | -47.5 | 4 | Not performed | Not performed |
| Example 7 | 49 | -15 | 3 | -40 | 4 | Not performed | Not performed |
| Example 8 | 49 | -15 | 3 | -40 | 4 | Not performed | Not performed |
| Example 9 | 242 | -12.5 | 2 | -47.5 | 4 | -25 | 2 |
| Example 10 | 242 | -12.5 | 2 | -47.5 | 4 | -25 | 2 |
| Example 11 | 242 | -15 | 2 | -47.5 | 4 | -25 | 2 |
| Example 12 | 242 | -12.5 | 2 | -47.5 | 4 | -25 | 2 |
| Example 13 | 40 | -20 | 2 | -41.5 | 4 | -25 | 2 |
| Comparative Example 1 | 49 | Not controlled | 3 | -40 | 4 | -25 | 2 |
| Reference Example 1 | 38 | -6 | 3 | -47.5 | 4 | Not performed | Not performed |

[Table 5]

| Formulation Example | Drying step | | | | | |
|---|---|---|---|---|---|---|
| | Primary drying | | | Secondary drying | | |
| | Temperature (°C) | Time (hr) | Vacuum pressure (Pa) | Temperature (°C) | Time (hr) | Vacuum pressure (Pa) |
| Example 1 | -10 | 80 | 10 | 60 | 8 | 10 |
| Example 2 | -20 | 140 | 10 | 60 | 8 | 10 |
| Example 3 | -10 | 80 | 10 | 60 | 8 | 10 |
| Example 4 | -10 | 80 | 10 | 60 | 8 | 10 |
| Example 5 | -10 | 80 | 10 | 60 | 8 | 10 |
| Example 6 | -10 | 80 | 10 | 60 | 8 | 10 |
| Example 7 | -20 | 110 | 10 | 60 | 8 | 10 |
| Example 8 | -10 | 80 | 10 | 60 | 8 | 10 |
| Example 9 | -10 | 80 | 10 | 60 | 8 | 10 |
| Example 10 | -10 | 80 | 10 | 60 | 8 | 10 |
| Example 11 | -10 | 80 | 10 | 60 | 8 | 10 |
| Example 12 | -10 | 80 | 10 | 60 | 8 | 10 |
| Example 13 | -10 | 60 | 10 | 60 | 8 | 10 |
| Comparative Example 1 | -20 | 145 | 10 | 60 | 8 | 10 |
| Reference Example 1 | -10 | 70 | 10 | 60 | 8 | 10 |

(Analysis method)

1) Specific surface area measurement method

The specific surface area of the lyophilized formulation was measured by a Brunauer-Emmett-Teller (BET) method using a specific surface area measuring apparatus (manufactured by Micromeriticis) according to the following procedure.

1. The lyophilized formulation was returned to room temperature in a glove box purged with nitrogen.
2. The lyophilized formulation was loosened in the glove box, and approximately 0.5 g of the lyophilized formulation was roughly weighed and added into a glass bottle.
3. The weighed value of an empty glass cell (including a cap) was recorded.
4. The lyophilized formulation roughly weighed was charged into the glass cell using a funnel, the glass cell was capped, and the weighed value was recorded.
5. Pretreatment was performed under conditions of 40°C and 1 hour using a degassing apparatus (manufactured by Micromeriticis).
6. Nitrogen gas was introduced into the sample as an adsorbent. At this time, the equilibration interval was set to 5 seconds. After the pretreatment, the temperature was returned to room temperature, and the weighed value was recorded.
7. This measurement was repeated three times.

2) Water content measurement method

The water content measurement of the lyophilized formulation was performed by the Karl Fischer (KF) method (volumetric titration method) according to the following procedure.

1. A specimen was transferred into a weighing bottle for water content measurement in a low-humidity glove box (relative humidity: 10% or less).
2. After measuring the weight, the specimen was added to a dehydrated solvent (mixed solution of methanol/formamide (70 : 30)), the solution was stirred, and it was confirmed that the specimen has been dissolved.
3. The weight of an empty weighing bottle was measured, and the water content was determined.
4. This measurement was repeated three times.

3) Reconstitution time measurement method

The reconstitution time of the lyophilized formulation was visually measured according to the following procedure.

1. 10 mL of water was added into the lyophilized formulation, and the vial was capped with a rubber stopper.
2. Immediately after the addition of water, the upper and lower surfaces of the vial were fixed and held, and the vial was shaken in the vertical direction of the vial.
3. A time from the start of shaking to the complete dissolution of the lyophilized formulation was recorded while visually checking whether there was any undissolved residue.
4. This measurement was repeated three times.

(Experimental results)

Table 6 shows the cooling temperature at the time of the step of cooling to a predetermined cooling temperature and introducing ice crystals, the average and standard deviation of the specific surface area ($m^2/g$) of three repeated measurements, the water content (%) and the reconstitution time (sec) of three repeated measurements.

As a result, the lyophilized formulations of Examples 1 to 13 all had a specific surface area of 0.6 to 1.1 $m^2/g$. The standard deviation of the specific surface area was also as small as 0.2 $m^2/g$ or less. In addition, the water content was 0.5% or less as the reference value, and the sample was dissolved in water in a short reconstitution time of 30 seconds or less. On the other hand, the specific surface area of the lyophilized formulation of Comparative Example 1 was larger than 1.1 $m^2/g$, and the water content was 0.5% or less as the reference value, but the reconstitution time was longer than 30 seconds. In addition, the specific surface area of the lyophilized formulation of Reference Example 1 was smaller than 0.6 $m^2/g$, and the water content was higher than 0.5% as the reference value. Therefore, in consideration of the water content and the reconstitution time of the lyophilized formulation, it was found that the specific surface area of the pharmaceutical formulation of the present invention is appropriately 0.6 to 1.1 $m^2/g$.

[Table 6]

| Formulation Example | Cooling temperature (°C) at the time of the step of cooling to a predetermined cooling temperature and introducing ice crystals | Specific surface area ($m^2/g$) | | Water content (%) | Reconstitution time (sec) |
|---|---|---|---|---|---|
| | | Average value | Standard deviation | | |
| Example 1 | -10 | 0.7442 | 0.09 | 0.38 | 9 |
| Example 2 | -10 | 1.0807 | 0.14 | 0.30 | 7 |
| Example 3 | -12.5 | 0.8742 | 0.10 | 0.32 | 12 |
| Example 4 | -12.5 | 0.9600 | 0.08 | 0.25 | 13 |
| Example 5 | -15 | 0.8566 | 0.04 | 0.28 | 10 |
| Example 6 | -10 | 0.8727 | 0.08 | 0.25 | 13 |
| Example 7 | -15 | 0.8947 | 0.05 | 0.24 | 17 |
| Example 8 | -15 | 0.8334 | 0.11 | 0.30 | 17 |
| Example 9 | -12.5 | 0.6934 | 0.07 | 0.39 | 10 |
| Example 10 | -12.5 | 0.9512 | 0.06 | 0.37 | 11 |
| Example 11 | -15 | 0.9363 | 0.06 | 0.28 | 22 |
| Example 12 | -12.5 | 0.9319 | 0.02 | 0.36 | 14 |

(continued)

| Formulation Example | Cooling temperature (°C) at the time of the step of cooling to a predetermined cooling temperature and introducing ice crystals | Specific surface area ($m^2/g$) | | Water content (%) | Reconstitution time (sec) |
|---|---|---|---|---|---|
| | | Average value | Standard deviation | | |
| Example 13 | -20 | 0.8105 | 0.02 | 0.26 | 20 |
| Comparative Example 1 | Not performed | 1.2815 | 0.09 | 0.17 | 48 |
| Reference Example 1 | -6 | 0.5040 | 0.06 | 0.54 | 10 |

4. Lyophilized formulation manufactured by lyophilizer (production machine) (1) Lyophilizer and other equipment

As the lyophilizer, the ice fog system for generating ice fog and the wireless product temperature sensor for measuring the product temperature, those listed in Table 7 were used.

[Table 7]

| Equipment/device name | Model | Manufacturer |
|---|---|---|
| Lyophilizer | RL-4536BS | Kyowa Vacuum Engineering Co., Ltd |
| Ice fog system | VERISEQ® Nucleation | IMA LIFE |
| Wireless product temperature sensor | TEMPRIS LAB | IQ-mobil |

(2) Components and composition of lyophilized formulation

The components and composition (per one vial) of the lyophilized formulation comprising the sodium salt of the compound represented by Formula (II), sodium p-toluene sulfonate, and sodium sulfate are as shown in Table 3.

(3) Formulation solution preparation method

First, 1,000 mg of Crystal form I was weighed in terms of the compound represented by Formula (I), and these crystals were added into a vial bottle and suspended in water for injection. The pH of the suspension was adjusted to 5.7 to 6 with 12% sodium hydroxide solution, and the crystals were dissolved as the sodium salt of the compound represented by Formula (II). Then, 900 mg of sucrose (manufactured by Merck) and 216 mg of sodium chloride (manufactured by Merck) were added to this solution. After stirring and dissolution, water for injection was added to the solution. The concentration of the solution was adjusted to 10 wt% in terms of the compound represented by Formula (I) and the pH was adjusted to 5.5 to 6. Thereafter, the solution was sterile filtered and the formulation solution was prepared. The formulation solution was filtered through a PVDF membrane filter having a pore size of 0.2 μm. A 14 mL clear glass vial that has been sterilized by dry heat was filled with 10 g of the filtrate, and the vial was semi-stoppered with a sterilized rubber stopper and then loaded into a lyophilizer.

(5) Manufacturing conditions of lyophilized formulation

Among the manufacturing conditions of the lyophilized formulation, the cooling conditions are shown in Table 8, and the drying conditions are shown in Table 9. The manufacturing of the lyophilized formulation is performed by 1) a step of cooling a liquid comprising the compound represented by Formula (I) or its pharmaceutically acceptable salt in a chamber of a lyophilizer to a predetermined cooling temperature, 2) a step of introducing ice crystals into the chamber, 3) a step of further cooling, 4) a step of heating and maintaining at the glass transition temperature or higher, 5) a primary drying step, and 6) a secondary drying step. In Table 8, the "step of cooling to a predetermined cooling temperature and introducing ice crystals" refers to the above steps 1) and 2), the "step of further cooling" refers to the above step 3), and the "step of heating and maintaining at the glass transition temperature or higher" refers to the above step 4). In Table 9, the "primary drying step" refers to the above step 5), and the "secondary drying step" refers to the above step 6).

[Table 8]

| Formulation Example | Manufacturing scale (number of vials) | Step of cooling to a predetermined cooling temperature and introducing ice crystals | | Step of further cooling | | Step of heating and maintaining at the glass transition temperature or higher | |
|---|---|---|---|---|---|---|---|
| | | Temperature (°C) | Time (hr) | Temperature (°C) | Time (hr) | Temperature (°C) | Time (hr) |
| Example 14 | 11000 | -12.5 | 1 | -47.5 | 4 | -25 | 2 |
| Reference Example 2 | 144 | -8 | 2 | -47.5 | 4 | -25 | 2 |

[Table 9]

| Formulation Example. | Drying step | | | | | |
|---|---|---|---|---|---|---|
| | Primary drying | | | Secondary drying | | |
| | Temperature (°C) | Time (hr) | Vacuum pressure (Pa) | Temperature (°C) | Time (hr) | Vacuum pressure (Pa) |
| Example 14 | -10 | 85 | 10 | 60 | 8 | 10 |
| Reference Example 2 | -10 | 80 | 10 | 60 | 8 | 10 |

(Analysis method)

The specific surface area measurement method, the water content measurement method, and the reconstitution time measurement method are the same as the analysis methods of the lyophilized formulation prepared by the lyophilizer (laboratory machine).

(Experimental results)

Table 10 shows the cooling temperature at the time of the step of cooling to a predetermined cooling temperature and introducing ice crystals, the average and standard deviation of the specific surface area ($m^2/g$) of three repeated measurements, the water content (%) and the reconstitution time (sec) of three repeated measurements.

As a result, the lyophilized formulation of Example 14 had specific surface areas of 0.8412 $m^2/g$ and 0.6 to 1.1 $m^2/g$. The standard deviation of the specific surface area was also as small as 0.2 $m^2/g$ or less. In addition, the water content was 0.5% or less as the reference value, and the sample was dissolved in water in a short reconstitution time of 30 seconds or less. On the other hand, the specific surface area of the lyophilized formulation of Reference Example 2 was smaller than 0.6 $m^2/g$, and the water content was higher than 0.5% as the reference value. Therefore, in consideration of the water content and the reconstitution time of the lyophilized formulation, it was found that the specific surface area of the pharmaceutical formulation of the present invention is appropriately 0.6 to 1.1 $m^2/g$.

[Table 10]

| Formulation Example | Cooling temperature (°C) at the time of the step of cooling to a predetermined cooling temperature and introducing ice crystals | Specific surface area ($m^2/g$) | | Water content (%) | Reconstitution time (sec) |
|---|---|---|---|---|---|
| | | Average value | Standard deviation | | |
| Example 14 | -12.5 | 0.8412 | 0.07 | 0.40 | 14 |
| Reference Example 2 | -8 | 0.3896 | 0.14 | 0.66 | 11 |

A relationship between the specific surface area and the water content of the lyophilized formulation manufactured by the manufacturing method of the present invention is shown in Fig. 1. As a result, it became clear that the water content tended to decrease as the specific surface area of the lyophilized formulation increased. When the specific surface area of the lyophilized formulation was 0.6 $m^2/g$ or more, the water content was 0.5% or less.

[INDUSTRIAL APPLICABILITY]

The specific surface area of the lyophilized formulation comprising the compound represented by Formula (I) or its pharmaceutically acceptable salt, or the compound represented by Formula (II) or its pharmaceutically acceptable salt (particularly, a sodium salt) is optimized by the manufacturing method of the present invention, whereby the water content in the lyophilized formulation and the reconstitution time could be controlled.

## Claims

1. A method for manufacturing a lyophilized formulation, which lyophilizes a liquid comprising a compound represented by Formula (I):

[Chemical formula 1]

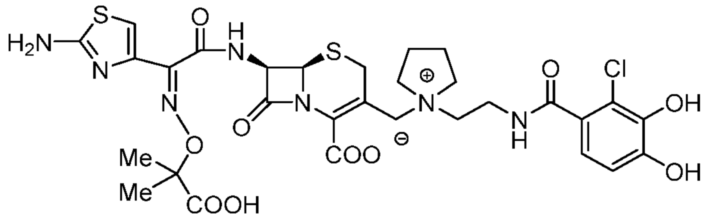

(I)

or its pharmaceutically acceptable salt, the method comprising at least the following steps:

step 1) cooling the liquid comprising the compound represented by Formula (I) or its pharmaceutically acceptable salt in a chamber of a lyophilizer to a predetermined cooling temperature, and
step 2) introducing ice crystals into the chamber.

2. The method for manufacturing a lyophilized formulation according to claim 1, which comprises the following steps after the step 2):

step 3) further cooling,
step 4) heating and maintaining at a glass transition temperature or higher, and
step 5) drying.

3. The method for manufacturing a lyophilized formulation according to claim 1 or 2, wherein the liquid comprises at least the following components:

a) the compound represented by Formula (I) or its pharmaceutically acceptable salt,
b) one or more selected from the group consisting of alkali metal chloride, alkali earth metal chloride, transition metal chloride and magnesium chloride, and
c) sugar and/or sugar alcohol.

4. The method for manufacturing a lyophilized formulation according to claim 1 or 2, wherein the liquid comprises at least the following components:

a) the compound represented by Formula (I) or its pharmaceutically acceptable salt,
b) sodium chloride, and
c) sucrose.

5. The method for manufacturing a lyophilized formulation according to any one of claims 1 to 4, wherein the step 1) is a step of cooling the liquid to -30°C to -5°C.

6. The method for manufacturing a lyophilized formulation according to any one of claims 1 to 4, wherein the step 1) is a step of cooling the liquid to -22°C to -10°C.

7. The method for manufacturing a lyophilized formulation according to any one of claims 1 to 6, wherein the step 2) is a step of introducing ice crystals.

8. The method for manufacturing a lyophilized formulation according to any one of claims 2 to 7, wherein the step 5) is a primary drying step and a secondary drying step.

9. The method for manufacturing a lyophilized formulation according to claim 8, wherein a time of the primary drying step in the step 5) is 100 hours or less.

10. A lyophilized formulation which comprises the compound represented by Formula (I) or its pharmaceutically acceptable salt, manufactured by the manufacturing method according to any one of claims 1 to 9.

11. The lyophilized formulation according to claim 10, wherein a specific surface area of the lyophilized formulation is 0.6 to 1.1 $m^2/g$.

12. A lyophilized formulation which comprises a compound represented by Formula (I):

[Chemical formula 2]

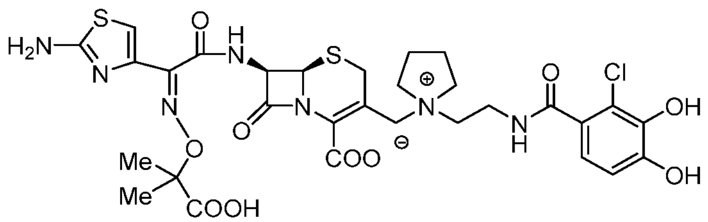

(I)

or its pharmaceutically acceptable salt, wherein a specific surface area of the lyophilized formulation is 0.6 to 1.1 $m^2/g$.

13. The lyophilized formulation according to claim 11 or 12, wherein a standard deviation of the specific surface area of the lyophilized formulation is 0.2 $m^2/g$ or less.

14. The lyophilized formulation according to any one of claims 11 to 13, wherein a reconstitution time of the lyophilized formulation is 30 seconds or less.

15. The lyophilized formulation according to any one of claims 11 to 14, wherein a water content of the lyophilized formulation is 0.5% or less.

16. The method for manufacturing a lyophilized formulation according to any one of claims 1 to 9, wherein the compound represented by Formula (I) or its pharmaceutically acceptable salt is an amorphous sodium salt of a compound represented by Formula (II):

[Chemical formula 3]

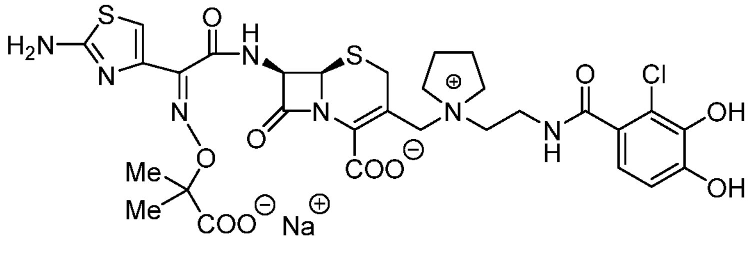

(II)

.

17. The lyophilized formulation according to any one of claims 10 to 15, wherein the compound represented by Formula (I) or its pharmaceutically acceptable salt is an amorphous sodium salt of the compound represented by Formula (II).

Fig. 1

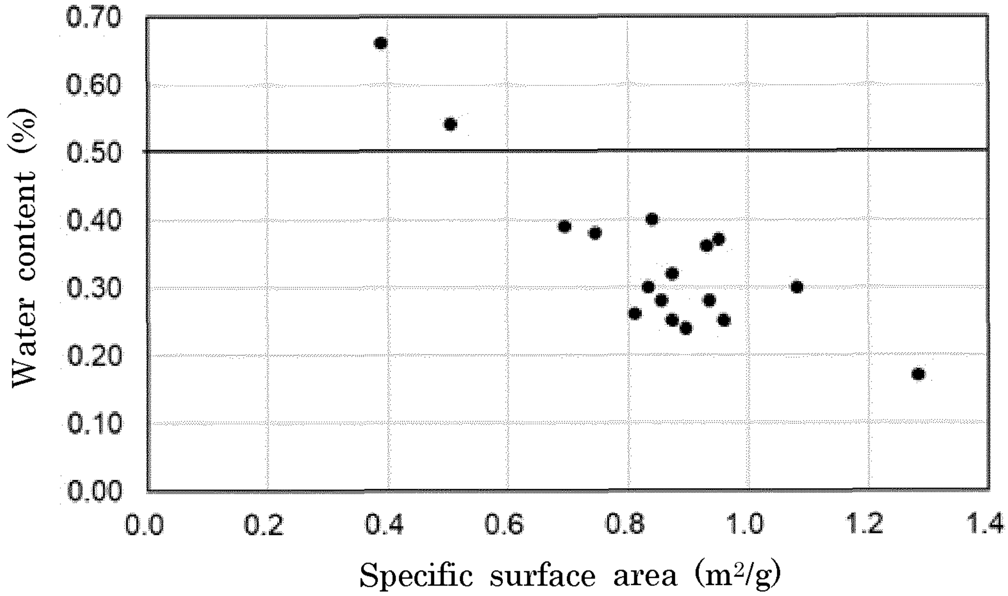
0.70
0.60
0.50
0.40
0.30
0.20
0.10
0.00
Water content (%)
0.0
0.2
0.4
0.6
0.8
1.0
1.2
1.4
Specific surface area (m2/g)

**INTERNATIONAL SEARCH REPORT**

International application No.
PCT/JP2021/027816

A. CLASSIFICATION OF SUBJECT MATTER
A61K 31/546(2006.01)i; A61K 9/19(2006.01)i; A61K 47/02(2006.01)i; A61K 47/26(2006.01)i; A61P 31/04(2006.01)i
FI: A61K31/546; A61P31/04; A61K9/19; A61K47/02; A61K47/26

According to International Patent Classification (IPC) or to both national classification and IPC

B. FIELDS SEARCHED

Minimum documentation searched (classification system followed by classification symbols)
A61K31/546; A61K9/19; A61K47/02; A61K47/26; A61P31/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2016/035846 A1 (SHIONOGI & CO., LTD.) 10 March 2016 (2016-03-10) paragraphs [0009]-[0010], [0052] | 1-17 |
| Y | 川崎英典，製剤と粒子設計 生産機での凍結乾燥プロセス開発期間を最小化するスケールアップ手法と氷核コントロールによる頑健な凍結乾燥プロセス獲得への挑戦，Pharm Tech Japan, 2015, 31(15), 2913-2921, page 2913, left column, page 2918, right column, (KAWASAKI, Hidenori, "Scale-up procedure to minimize the time necessary to develop commercial freeze-drying cycles and challenge to acgieve more robust freeze-drying process by controlling ice nucleation") | 1-17 |
| Y | JP 2019-85338 A (TEVA TAKEDA PHARMA LTD.) 06 June 2019 (2019-06-06) claim 13, paragraphs [0030]-[0034] | 1-17 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 September 2021 (09.09.2021) | 21 September 2021 (21.09.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/027816

| Patent Documents referred in the Report | Publication Date | Patent Family | Publication Date |
|---|---|---|---|
| WO 2016/035846 A1 | 10 Mar. 2016 | US 2017/0281639 A1 paragraphs [0023]-[0063], [0187] EP 3189841 A1 | |
| JP 2019-85338 A | 06 Jun. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010050468 A
- WO 2016035846 A
- JP 2014512510 A
- WO 2016035845 A
- WO 2016035847 A


### Non-patent literature cited in the description


- *Journal of the Japan Society of Pharmaceutical Machinery and Engineering,* 2015, vol. 24 (2), 39-51